# EUROPEAN PATENT SPECIFICATION

(11) **EP 3 640 330 B1**
(45) Date of publication and mention of the grant of the patent: **08.12.2021**
(21) Application number: 18200482.0
(22) Date of filing: 15.10.2018
(51) Int. Cl.: C12N 15/10, C12Q 1/6806

(54) **METHOD FOR SEQUENTIAL ANALYSIS OF MACROMOLECULES**
VERFAHREN ZUR SEQUENTIELLEN ANALYSE VON MAKROMOLEKÜLEN
PROCÉDÉ D'ANALYSE SÉQUENTIELLE DE MACROMOLÉCULES

(43) Date of publication of application: 22.04.2020
(73) Proprietor: Consiglio Nazionale Delle Ricerche, 00185 Roma (IT); Istituto Nazionale Di Genetica Molecolare-INGM, 20122 Milano (IT); IFOM Fondazione Istituto Firc di Oncologia Molecolare, 20139 Milano (IT)
(72) Inventor: LANZUOLO, Chiara, 20900 Monza (IT); MARULLO, Fabrizia, 00152 Roma (IT); LUCINI, Federica, 22100 Como (IT); FERRARI, Francesco, 20141 Milano (IT); SEBESTYÉN, Endre, 1089 Budapest (HU)
(74) Representative: Turri, Elisa

(56) References cited:
- WO-A1-2010/065266
- WO-A1-2017/136198
- US-A1- 2008 241 845
- Sheila S. Teves ET AL: "Salt Fractionation of Nucleosomes for Genome-Wide Profiling" In: "Methods in Molecular Biology", 1 January 2012 (2012-01-01), Humana Press, Inc., US, XP055561567, ISSN: 1064-3745 vol. 833, pages 421-432, DOI: 10.1007/978-1-61779-477-3_25, * the whole document * * *
- S. HENIKOFF ET AL: "Genome-wide profiling of salt fractions maps physical properties of chromatin", GENOME RESEARCH, vol. 19, no. 3, 22 December 2008 (2008-12-22), pages 460-469, XP055561560, US ISSN: 1088-9051, DOI: 10.1101/gr.087619.108
- RENÉA M DIRKS ET AL: "Genome-wide epigenomic profiling for biomarker discovery", CLINICAL EPIGENETICS, BIOMED CENTRAL LTD, GB, vol. 8, no. 1, 21 November 2016 (2016-11-21), pages 1-17, XP021239783, ISSN: 1868-7075, DOI: 10.1186/S13148-016-0284-4
- E Rocha ET AL: "Differential salt fractionation of active and inactive genomic domains in chicken erythrocyte", Journal of Biological Chemistry, 10 July 1984 (1984-07-10), pages 8558-8563, XP055561565, United States Retrieved from the Internet: URL:http://www.jbc.org/content/259/13/8558 .full.pdf [retrieved on 2019-02-26]
- D. C. HE ET AL: "Core filaments of the nuclear matrix", THE JOURNAL OF CELL BIOLOGY : JCB, vol. 110, no. 3, 1 March 1990 (1990-03-01) , pages 569-580, XP055561983, US ISSN: 0021-9525, DOI: 10.1083/jcb.110.3.569
- CHRISTIN HERRMANN ET AL: "Differential Salt Fractionation of Nuclei to Analyze Chromatin-associated Proteins from Cultured Mammalian Cells", BIO-PROTOCOL, vol. 7, no. 6, 1 January 2017 (2017-01-01) , XP055561462, Sunnyvale, CA, USA ISSN: 2331-8325, DOI: 10.21769/BioProtoc.2175

## Description

### FIELD OF THE INVENTION

The present invention relates to a new sequencing method, herein called "SAMMY-seq" (Sequential Analysis of MacroMolecules accessibilitY), able to detect chromatin changes. SAMMY-seq technology can identify a specific chromatin state (heterochromatin and/or euchromatin) and further provide data on its accessibility. The present method may find application in detecting alterations in chromatin structure and functions that may lead to developmental defects or pathologies. Therefore, the method may be applied in diagnosis of pathologies that alter genome structure, as cancer or laminopathies. The present invention also refers to a kit for analysis of genomic accessibility on a small number of cells, that may be used in biomedical field e.g. for diagnosis of the above defined pathologies.

### BACKGROUND TO THE INVENTION

Nuclei of higher organisms are complex structures that encapsulate the genome in a small physical space. In spite of first observations, describing DNA as an unshaped mass in interphasic nuclei, scientists demonstrated that each chromosome is folded in a highly organized fashion and occupies specific territories in the nuclear space. Indeed, chromatin is non-homogenously distributed inside the nucleus, as shown by electron microscopy. In particular, most of the darkly stained chromatin (heterochromatin) is found around the nucleoli or at the nuclear periphery interrupted with euchromatin at nuclear pores or in the nucleus interior. This non―randomic genome positioning ensures adequate regulation of gene expression and maintenance of genome integrity. Epigenetic mechanisms, acting at different levels from DNA up to the higher order chromatin structures, ensure the correct folding and positioning of the genome, finely regulating the transcriptional output. To achieve the higher order configurations the genetic material utilizes macroscopic structures of the nucleus, such as the nuclear pores and the nuclear lamina as spatial references. Thus, some epigenetic factors cooperate with nuclear structures to fold and to maintain the steady state of chromatin conformation. Heterochromatin is a peculiar part of the genome, characterized by highly compact chromatin architecture and specific nuclear compartmentalization². Alterations in heterochromatin structure and functions can lead to developmental defects and increasing evidence suggest that the correct heterochromatin conformation is a hallmark of healthy cells. In line with these findings, it has been shown that in several human diseases dysfunctional heterochromatin acquires an aberrant shape and distribution. In recent years the study of heterochromatin aroused the interest of the scientific community and technologies able to characterize this part of the genome are in great demand. At the moment, scientists can analyze heterochromatin by using ChIP-seq for specific heterochromatin marks (H3K9me2,3 and H3K27me3) or Lamin to identify Lamin Associated Domains (LADs), enriched of constitutive heterochromatin. The Patent Application US2016168622 refers to immediate chromatin immunoprecipitation procedure ("ZipChIP"), reducing the time and increasing sensitivity allowing for rapid screening of multiple loci. ZipChIP enables the detection of histone modifications (e.g., H3K4 mono- and trimethylation) and at least two yeast histone demethylases, Jhd2 and Rph1, which were previously found difficult to detect using standard methods. ZipChIP further relates to the enrichment of the histone deacetylase Sir2 at heterochromatin in yeast and enrichment of the chromatin remodeler, PICKLE, in Arabidopsis thaliana. However, ChIP procedure is dependent on antibody efficiency, batch variability and samples are pretreated with chemical modifications that can alter the genome conformation. In addition, ChIP technology provides only 1D data, describing the linear distribution of heterochromatin marks or Lamin proteins on the genome. Finally, ChIP samples are mostly treated with crosslinking reagents that chemically modify the chromatin causing high background after sequencing ^{52, 53}. The majority of commercial Lamin antibodies are not ChIP grade and Lamin ChIP-seq performed in all lamin associated pathologies (laminopathies) does not ensure a comparable efficiency in the recognition by the antibodies of the wild type and mutated form of the Lamin protein. To overcome the absence of Lamin ChIP grade antibody, in the past, DamID has been frequently used to map lamina-associated domains (LADs). However, this technology relies on a step of transfection with the construct containing the protein of interest fused to the Escherichia coli DNA adenine methyltransferase (Dam). This renders the technology not suitable on primary cultures or tissues. HiC technology can provide information on the entire genome conformation. However, there are limitations in the high number of cells required for the assay and the number of reads necessary for the data analysis. Recent techniques DNAse-seq and ATAC-seq for mapping chromatin accessibility and highlight transcribed chromatin domains are able to identify euchromatin. The Patent Application WO2010065266 refers to methods and compositions for determining accessibility of a DNA modifying agent in genomic DNA. The method comprises simultaneously permeabilizing or disrupting a cell membrane of a cell and contacting the cell with a DNA cleaving or modifying agent under conditions such that the agent cleaves or modifies the genomic DNA in the cell, wherein different regions of the genomic DNA are cleaved or modified to a different extent by the agent, thereby generating cleaved and intact DNA regions, or modified and unmodified DNA regions; and detecting a quantity of at least one intact or unmodified or modified DNA region or cloning, isolating, or nucleotide sequencing at least one intact or unmodified or modified DNA region. However, this technology does not provide information on heterochromatin and it does not quantitatively compare DNA presence in accessible vs inaccessible fractions. Herrmann C. et al., Bio Protoc.; 7 (6), 2017 refers to a procedure comprising saline extractions using the enzyme MNase to digest the DNA. This technology primarily analyze protein composition and does not recover nucleic acids. Present inventors, starting from the protocol described in ²⁹ and modifying it by adding some washing between extractions and increasing volumes to clean up fractions previously developed a method for chromatin fractionation ^{18, 55}. Therefore, it is still needed a method able to provide structural and quantitative genome wide information, said method being able to identify the chromatin state and further provide data on its accessibility, which overcomes the above disadvantages.-DNA accessibility often reflects the transcriptional state of the genome: the more accessible and less condensed euchromatin is generally enriched in expressed genes, whereas heterochromatin contains highly condensed DNA, including pericentromeric and telomeric regions³⁻⁵ and also genomic regions with unique packaging properties targeted by the Polycomb-group proteins (PcG)⁶. These are developmentally regulated transcriptional factors acting as parts of multimeric complexes such as PRC1 and PRC2⁷. Another key player of chromatin organization is the nuclear lamina (NL). The NL is mainly composed of A- and B-type lamins, intermediate filaments of 3.5 nm thickness which form a dense network extending throughout the nuclear space from the nuclear periphery to the nucleoplasm^{8,9}. The NL preferentially interacts with the genome at specific regions called Lamina Associated Domains (LADs), with sizes ranging from 100Kb to 10Mb¹⁰. LADs are enriched in H3K9me2 and H3K9me3 histone modifications that are typical of inactive heterochromatic regions. LAD borders are marked by the H3K27me3 histone mark¹¹⁻¹³, which is characteristic of inactive PcG-regulated chromatin regions. The ensemble of lamins, chromatin marks and PcG factors around LADs creates a repressive environment^{14,15}, with heterochromatin and PcG target regions adjacent to each other¹⁶. In line with these observations, inventors previously demonstrated that Lamin A functionally interacts with PcG¹⁷⁻¹⁹. A growing body of evidence suggests that LADs association or release from lamina are related to the spatiotemporal regulation of gene expression in cell differentiation processes^{13,20,21}. The crucial function of these dynamics is attested by an entire class of genetic diseases, called *laminopathies,* where specific components of the NL are altered^{13,22,23}. In recent years, the widespread adoption of experimental techniques based on high-throughput sequencing (NGS) has been instrumental in advancing the knowledge of chromatin structure and its alterations in disease²⁴. Notably, several of these techniques were originally designed to map active chromatin regions, such as ATAC-seq, DNase-seq and MNase-seq. On the other hand, few options are available for genome-wide mapping of heterochromatin and LADs. These include mapping heterochromatin associated histone marks (H3K9 methylation) by ChIP-seq and mapping LADs by ChIP-seq or DamID-seq. Such techniques suffer major limitations as ChIP-seq relies on crosslinking, causing technical biases, and antibodies, with several potential issues of sensitivity or cross-reactivity. Whereas DamID-seq relies on the exogenous expression of a transgene, thus it can't be applied on primary cells or tissues. Here, inventors present a novel high-throughput sequencing based technique to map lamina associated heterochromatin regions. The present technique is based on the sequential isolation and sequencing of multiple chromatin fractions, enriched for differences in accessibility. The present method is robust, fast, easy-to-perform, it does not rely on crosslinking reagents or antibodies, and it can be applied on primary cells. In normal human fibroblasts, the present technique can reliably identify genomic regions with heterochromatin marks (H3K9me3) and LADs. Thus, as a test case inventors applied it to characterize chromatin alterations in the early stages of a laminopathy named Hutchinson-Gilford progeria syndrome (HGPS, OMIM 176670). HGPS is a rare and fatal disease caused by a de novo point mutation in the LMNA gene²⁵, resulting in a truncated splicing mutant form of Lamin A protein, known as progerin. The disease phenotype is characterized by early onset of a complex set of symptoms causing severe premature aging in pediatric patients. At the cellular level, the accumulation of progerin induces progressive alterations of nuclear shape and cellular senescence²⁶. At the molecular level, LADs organization and PcG-dependent H3K27me3 mark are altered^{27,28}. Moreover, extensive disruption of chromatin structure has been reported by Hi-C in late-passage HGPS-derived fibroblasts, whereas this could not be detected in early passage cells, commonly considered a model for early stages of the disease²⁷.

Therefore a sensitive method able to provide structural and quantitative genome wide information, said method being able to identify the chromatin state and further provide data on its accessibility is still needed to detect early alterations of chromatin structure in order to diagnose or predict the risk of laminopathies, such as progeria syndrome.

Teves et al. (2012) discloses salt fractionation of nucleosomes for genome-wide profiling (Meth Mol Biol 833:421-432).

### BRIEF DESCRIPTION OF THE INVENTION

The invention is defined by the scope of the appended claims.

Inventors set up a novel technique based on high-throughput nucleic acid sequencing that classifies chromatin regions on the basis of their accessibility. Inventors called this procedure SAMMY-seq (Sequential Analysis of MacroMolecules accessibilitY). Notably, SAMMY-seq is the only one allowing genome-wide mapping of Lamina associated heterochromatin regions in primary tissue samples. SAMMY-seq allows documenting the heterochromatin state of subjects, who may be for example patients affected by laminopathies or cancer, without the use of crosslinking reagents and antibodies. The protocol is based on the sequential purification of different chromatin fractions, corresponding to increasingly compacted chromatin regions, whose distribution along the genome is then identified using high throughput sequencing. By comparison of different fractions, it is possible to quantitatively follow heterochromatin accessibility at specific genomic regions. Starting from the prior art protocols, inventors extracted DNA from fractions and treat it for sequencing, working on DNA purification and sonication. The added sonication steps are different for every different fraction and depend on cell type. The skilled man is able to adapt the sonication step to every kind of cell, based on his knowledge. The DNA contained in the different fractions is then subjected to sequencing. The inventors also added another fraction, allowing purifying smaller fragments that, in the prior art protocol, were oversonicated and discarded. In more compacted fractions the inventors preferentially enrich for H3K9me3-marked heterochromatin regions, with depletion instead for open chromatin marks, such as H3K4me1 histone marks. On the other hand, in more accessible fractions they enrich for H3K36me3-marked euchromatin. Moreover, the inventors can quantitatively follow heterochromatin accessibility by measuring the relative enrichment across distinct sequenced fractions. Alternatively, inventors extracted and sequenced RNA from chromatin fractions. This new technique constitutes a major breakthrough in the field, as it provides novel information complementary and not overlapping to other high-throughput based epigenomic techniques commonly used to study chromatin structure and function, as for example ChIP-seq for mapping chromatin marks, DamID-seq for mapping association to nuclear lamina, ATAC-seq or DNAse-seq for mapping chromatin accessibility. Sammy technology can identify a specific chromatin state (e.g. heterochromatin and/or euchromatin) mainly transcriptionally inactive and further provide data on the heterochromatin accessibility. At the moment technologies working in fixation-free genetic material an able to provide same information are not available. Furthermore, the present technique allows overcoming several major limitations of other epigenomic techniques, namely:
1) A small amount of cells (e.g. from 50.000-500.000 depending on the cell type) is required for this protocol, thus allowing the analysis of rare cell populations or primary cultures or tissues with limited amount of biological material;
2) The technique does not involve chemical modifications of chromatin, which might cause artefacts. In particular SAMMY-seq does not rely on crosslinking reagents, thus avoiding formaldehyde-based artefacts and high background after sequencing;
3) The system is not relying on antibodies for enriching specific chromatin fractions. This completely avoids problems related to antibodies specificity or cross-reactivity. This is particularly important when studying chromatin changes in cells where protein levels of chromatin factors could be altered, thus allowing more flexibility in terms of experimental design compared to antibody-based techniques, or in cells presenting a mutated form of protein of interest, not equally recognized by the antibody;
4) The SAMMY technology can be used instead of Lamin A ChIP-seq. In the S4 fraction inventors found on average 79% of Lamin Associated Domains (LADs). This is relevant because at the moment commercially available Lamin A antibodies do not allow reliable mapping of LADs.
5) The analysis of SAMMY-seq, relying on pairwise comparisons between fractions extracted from the same population, can be used for quantitative analysis of heterochromatin accessibility.
6) The study of DNA and RNA extracted from the same fractions allows direct correlation between chromatin accessibility and transcript localization. This can be used to localize long noncoding RNA on the chromatin and to investigate their functions.

The present technology is complementary to recent technologies, DNAse-seq and ATAC-seq, able to identify euchromatin and not the less accessible part of the chromatin which is instead isolated with the present method. In addition, the SAMMY-seq, having more than one fraction allows a stratification of chromatin accessibility, providing a quantitative method to finely detect euchromatin/heterochromatin conformational changes. The present new high-throughput sequencing-based technique, named SAMMY-seq, for genome-wide mapping of chromatin regions enriched by differential accessibility allows in particular to analyze Hutchinson-Gilford progeria syndrome (HGPS) skin fibroblasts and normal control fibroblasts. Using SAMMY-seq in wild type fibroblasts inventors reliably map lamina associated heterochromatic regions. To test the sensitivity of the present technology to chromatin structure alterations, inventors analyzed Hutchinson-Gilford progeria syndrome fibroblasts. This syndrome is characterized by the progressive accumulation of progerin, a mutated form of Lamin A, which leads to chromatin structure disruption, by interfering with lamina associated domains. In progeria fibroblasts, inventors were able to detect early stage changes in chromatin accessibility. Of note, early alterations of chromatin do not modify the distribution of the heterochromatin mark H3K9me3, but are associated with transcriptional deregulation of Polycomb target genes. The present results show that SAMMY-seq is a versatile and sensitive tool to analyze heterochromatin regions.

### DETAILED DESCRIPTION OF THE INVENTION

Here inventors present a novel technique based on high-throughput nucleic acid sequencing useful to isolate and map chromatin regions with different levels of accessibility, without the use of crosslinking reagents and antibodies. Inventors named the present technology SAMMY-seq (Sequential Analysis of MacroMolecules accessibilitY), as a tribute to Sammy Basso, the founder of the Italian Progeria Association. Inventors in particular applied SAMMY-seq ON early-passage HGPS fibroblasts and inventors found HGPS patient-specific changes of chromatin accessibility. Although HGPS chromatin remodeling is not accompanied by H3K9me3 changes, differentially compacted domains affect the transcriptional state of PcG regulated bivalent genes. These findings demonstrate the sensitivity of the present technique and suggest that chromatin structural changes are an early event in HGPS nuclear remodeling that interferes with proper PcG control. Disclosed herein is an in vitro method for analyzing nucleic acids, the method comprising:
a) providing at least one isolated cell that comprises genetic material and
b) performing a salt-based sequential fractionation of isolated genetic material to separate genomic regions on the basis of their accessibility and obtain chromatin fractions and
c) extracting the nucleic acids from the above chromatin fractions and
d) optionally sonicating the extracted nucleic acids such that chromatin is fragmented to have comparable profile of different fractions and
e) sequencing the extracted nucleic acids of step c) or the sonicated nucleic acid of step d).

The nucleic acid is DNA or RNA. When the nucleic acid is RNA, step d) is not performed.

Preferably the nucleic acids are chromosomal. Step c) preferably comprises also purification of the nucleic acids.

The method may comprise before step b) a further step of isolating genetic material from the cell.

In the context of the present invention the term "accessibility" refers to the accessibility to the used enzyme. E.g., a nucleic acid which is less compact is less accessible while a nucleic acid less accessible is more compact. Preferably, the cell of step a) is previously obtained by:
- collecting cell removed from the culture substrate, preferably by trypsinization or
- digesting fragments of tissue, preferably in a dispase/collagenase buffer, in order to obtain a cell suspension and by filtering said suspension and collecting filtered cells.

Preferably, the at least one cell of step a) is a population of cells comprising 5×10⁴-50×10⁵, more preferably 5-50×10⁵ cells. However, the number of the cells may vary depending on the cell type used. E.g. for lymphocytes at least 1.5×10⁶ cells may be used, while with prostate tissue 5×10⁴ may be used.

Preferably, step b) comprises obtaining the following fractions: a fraction enriched of soluble nuclear and cytoplasmic proteins ("S1"), a fraction enriched in chromatin binding protein ("S2"), a fraction enriched in histones and histone bound proteins ("S3"), a fraction enriched in nucleo-skeleton and membrane associated proteins ("S4").

Only in case of DNA extraction, the fraction S2 is sub fractionated in two sub fractions and one sub fraction comprising smaller nucleic acid fragments of about 100-200bp ("S2small" or "S2s") is directly isolated while the other sub fraction ("S2larger" or "S2l") is subjected to the sonication of step d).

Preferably, step b) comprises the following steps:
- extraction and solubilization of proteins from the cell to obtain the S1 fraction;
- nucleic acid digestion to obtain the S2 fraction;
- high salt extraction to obtain the S3 fraction and
- urea extraction to obtain S4 fraction and optionally
- sub fractioning S2 fraction into at least two fractions.

The above extraction of step b) is preferably carried out by Triton extraction.

The above nucleic acid digestion of step b) is preferably carried out by DNAse treatment.

The sub-fractioning of S2 is preferably carried out after extraction (and optionally purification of DNA). It is preferably carried out by means of a size-based DNA separation.

Preferably step b) comprises the following steps:
i. permeabilization of cell membranes and extractions of proteins from the cell to solubilize cytosolic and nuclear soluble proteins to obtain a solution, centrifugation of the obtained solution to separate cytoskeletal frameworks from soluble proteins thus obtaining a supernatatant and a pellet and collection of the obtained supernatant thus obtaining fraction S1, and/or
ii. resuspension of the pellet obtained in step i), incubation of the suspended pellet with a nucleic acid cleaving agent to digest the nucleic acid, addition of a buffer able to stop the digestion, (preferably (NH4)2SO4 when using DNAse) centrifugation to obtain a supernatant and a pellet and collection of the supernatant thus obtaining fraction S2; and/or
iii. resuspension of the pellet obtained in step ii) in a buffer removing the histones from nucleic acid, centrifugation to obtain a supernatant and a pellet and collection of the supernatant thus obtaining fraction S3; and/or
iv. resuspension of the pellet obtained in step iii) in a buffer removing the histones from nucleic acid, centrifugation to obtain a supernatant and a pellet, suspension of the pellet in a solution able to dissolve cellular membranes releasing membrane associated proteins, preferably said solution being urea, collection of the supernatant thus obtaining fraction S4.

More preferably step i) is carried out by incubation of the cell in a cytoskeleton buffer supplemented with a nonionic detergent, more preferably selected from the group of Triton X-100, NP40, Tween20 and/or wherein
step ii) comprises:
   - washing the pellet obtained after centrifugation of i) in a cytoskeleton Triton buffer, centrifugation of the obtained suspension, collection and resuspension of the obtained pellet in a cytoskeleton buffer;
   - addition of a DNA cleaving agent under conditions such that the agent cleaves the genomic DNA in the cell to solubilizes chromatin;
   - addition of ammonium sulphate to obtain a solution;
   - centrifugation of the solution obtained and collection of the obtained supernatant to have S2 fraction; and/or wherein
step iii) comprises:
   - washing the obtained pellet in CSK buffer to obtain a solution;
   - centrifugation of the obtained solution to obtain a supernatant and a pellet;
   - salt extraction of the pellet, preferably by resuspension in CSK NaCl buffer, more preferably 2M NaCl in CSK;
   - centrifugation of the solution obtained and collection of the obtained supernatant to have S3 fraction; and/or wherein
step iv) comprises:
   - washing the obtained pellet in a buffer, preferably CSK NaCl, more preferably 2M NaCl in CSK, for at least two times;
   - centrifugation of the obtained solution to obtain a pellet;
   - solubilize the pellet in urea buffer to have S4 fraction.

Preferably, the nucleic acid cleaving agent is an enzyme, preferably selected from a DNase or a restriction enzyme, more preferably RNase-free, preferably in a CSK buffer.

Preferably, only for DNA extraction, in step d) the fractions S2l and/or S3 and/or S4 are sonicated. In the RNA extraction there is no need of a sonication step as the nucleic acid is directly extracted from the fractions.

In the method disclosed herein, after the fractioning of step b) the DNA may be extracted. In this case the fraction S2 can be sub fractionated as described above and, typically, the DNA is not extracted by the fraction S1. Alternatively, after step b), the RNA may be extracted. In this case the RNA is extracted also by the fraction S1.

Preferably the at least one cell is selected from the group of: eukaryotic cells, prokaryotic cells bacteria cells, plant cells, fungal cells, and animal cells, preferably human cell.

In a preferred embodiment the method further comprises comparing the obtained sequence with a control for screening of patients affected by a pathology that alter genome conformation or for diagnosis of a pathology that alter genome conformation, preferably cancer or laminopathy, more preferably progeria, even more preferably Hutchinson-Gilford progeria syndrome, or aging or pathologies correlated to aging.

A further object of the invention is an in vitro method of identifying cells suffering from a pathology by determining chromatin conformation of the target loci known to be associated with the disease by treating the group of cells according to above defined method. The present method indeed allows to obtain information on chromatin conformation, which is normally associated with a specific histone methylation. Further disclosed is the use of a kit for analyzing chromosomal nucleic acids comprising at least one of the following: a high salt solution, reagents for isolating genetic material from cells, reagents for purifying genetic material, one or more restriction enzyme or a DNase, reagents for PCR amplifying target loci of interest, and reagents for sequencing the amplified target loci of interest using a DNA or RNA sequencing platforms. The above high salt solution is preferably a solution able to remove the histones, preferably a ionic salt, more preferably it is NaCl. Another object of the invention is the use of the above defined kit for carrying out the above defined method.

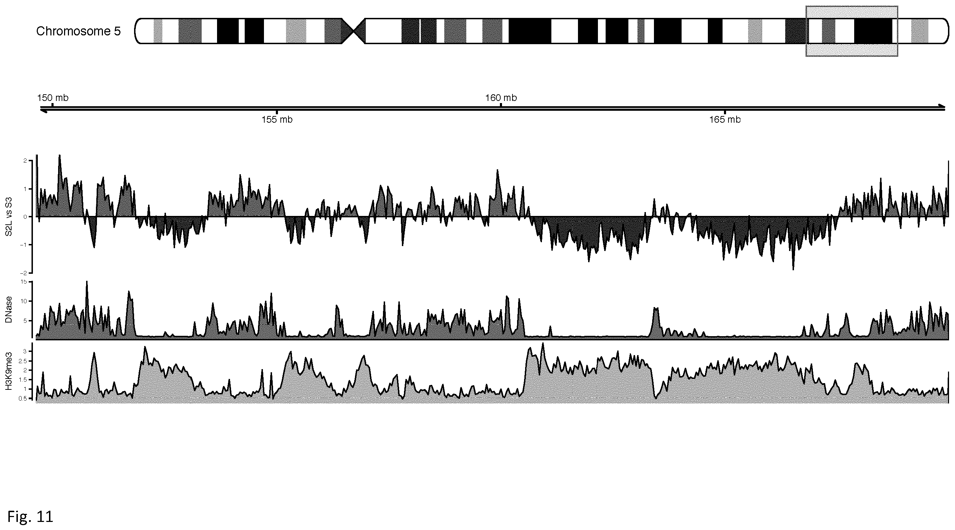

Step c) of the above method is preferably carried out, after fractionation, on S2 (or their subfractions), S3, S4 fractions and/or performed, for DNA extraction, by phenol/chloroform extraction, preferably after incubation with RNAseA and Proteinase K. Alternatively, if the nucleic acid that has to be analyzed is RNA, the RNA extraction of the above step c) is preferably carried out, after fractionation, on S1, S2, S3, S4 fractions, preferably by using available commercial kit, such as Maxwell RSC miRNA Tissue Kit AS 1460. The method according to the invention was successfully tested on different cells and tissue, in particular on human fibroblasts, murine myoblasts, human lymphocytes and human prostatic biopsies. The method according to the invention allows to obtain fractions that may be compared to each other and allows to get information by the sequences to differentiate the chromatin states. Moreover, once obtained the sequences, they can be analyzed for the presence of deletions and/or translocations. The present method indeed allows to analyze the sequence to determine the chromatin structure of target loci. After having obtained the different fractions, not all of them must be necessary sequenced. However, it is necessary to sequencing at least two fractions in order to obtain the ratio a posteriori, necessary for the method to be quantitative. For obtaining the S4 fraction it is not performed a centrifugation, in order to not loose DNA potentially still bound to not digested membranes. The sub fractioning of S2 allows to map the chromatin which is more open. Indeed, as shown in figure 11, the fraction S2l correlates with the euchromatin (open chromatin). Sequencing is preferably performed by high-throughput sequencing platform, even more preferably using Illumina HiSeq2500 or HiSeq 2000. For sequencing, DNA libraries or cDNA libraries (in case of analysis of RNA) are previously prepared from the extracted (and optionally sonicated) nucleic acids and then the DNA or the RNA is sequenced.

Further disclosed is a method of determining the differences in the chromatin structure of target loci between a first group of cells and a second group of cells, the method comprising:
a. determining chromatin structure of the target loci in the first group of cells by treating the first group of cells according to above disclosed method,
b. determining chromatin structure of the target loci in the second group of cells by treating the second group of cells according to the above disclosed method, and
c. comparing the chromatin structure obtained in steps a and b to determine the differences in the chromatin structure (and optionally in the methylation state) of the target loci between the first group of cells and the second group of cells.

Further disclosed is a method of identifying genes associated with a pathology, the method comprising:
a. determining chromatin structure of the promoters and/or other loci of a set of genes in normal cells by treating the normal cells according to the above disclosed method,
b. determining chromatin structure of the promoters and/or other loci of the set of genes in cells suffering from the condition by treating the cells suffering from the condition according to the above disclosed method, and
c. comparing the chromatin structure of promoters of the genes in the normal cells and the cells suffering from the condition to identify genes associated with the pathology.

The number of cells that may be analyzed may be variable between 5-50^{∗}10⁵ cells. The analyzed cells may be also derived from tissue sample, e.g. derived from a cancer.

In a preferred embodiment, the SAMMY-seq procedure comprises a preliminary step of detaching and colleting cells. Cells are preferably subjected to trypsinization. Trypsin is then preferably inhibited by resuspending the cells with media containing serum. To collect the cells, it is preferably to avoid the use of scrapers in order to preserve cellular and nuclear structures and proteins compartmentalization. It is preferable to use cold PBS (preferably stored at 4°C) to wash cells to prevent proteins degradation. If tissues are used, they have to be firstly cut in small pieces and digested, preferably in a 2 mL of dispase/collagenase buffer, for preferably approximately 30-60 min, until a cell suspension is obtained. The suspension is then filtered preferably with a 50 µm filter and the cells collected.

The method according to the invention is preferably used for mapping lamina associated heterochromatic region called SAMMY-seq.

In a preferred embodiment of the present invention, the cell subjected to the method is a primary cell and/or it was not subjected to chemical modifications of chromatin.

In the context of the present invention the "control" may be the sequence of a nucleic acid (or a sample) obtained from a normal subject or a subject who is not affected by the pathology that has to be screened or diagnosed. The method according to the invention may applied to any nucleic acid, preferably to DNA or RNA.

The invention will be now illustrated by means of non-limiting examples referring to the following figures.

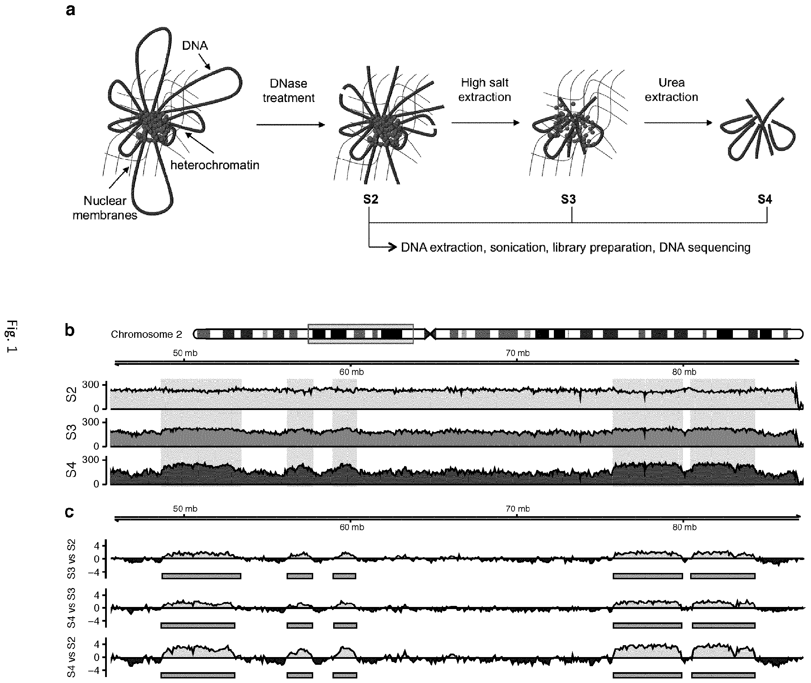

**Figure 1****. SAMMY-seq reliably isolates specific DNA regions in fibroblast samples. a,** Schematic representation of SAMMY-seq. Chromatin fractions are sequentially isolated after DNase treatment, high salt and urea buffers extractions. The associated genomic DNA is extracted, sonicated and processed for high-throughput sequencing. b, Distribution of SAMMYseq reads along a representative region (50Mb region in chr2:45570000-87200000). Library size normalized read counts over 10Kb genomic bins are shown for each sequenced fraction of a control fibroblast sample. c, Differential reads distribution across pairwise comparisons of SAMMY-seq fractions in a control fibroblast sample along the same genomic region as above. Less accessible fractions are compared to more accessible ones used as reference (S3 vs S2; S4 vs S3; S4 vs S2). Regions of signal enrichment or depletion over the reference samples are marked in yellow or purple, respectively. The smoothed differential signal is calculated with SPP, and significantly enriched regions (SAMMY-seq domains) are called with EDD and reported as grey boxes under the enrichment signal track.

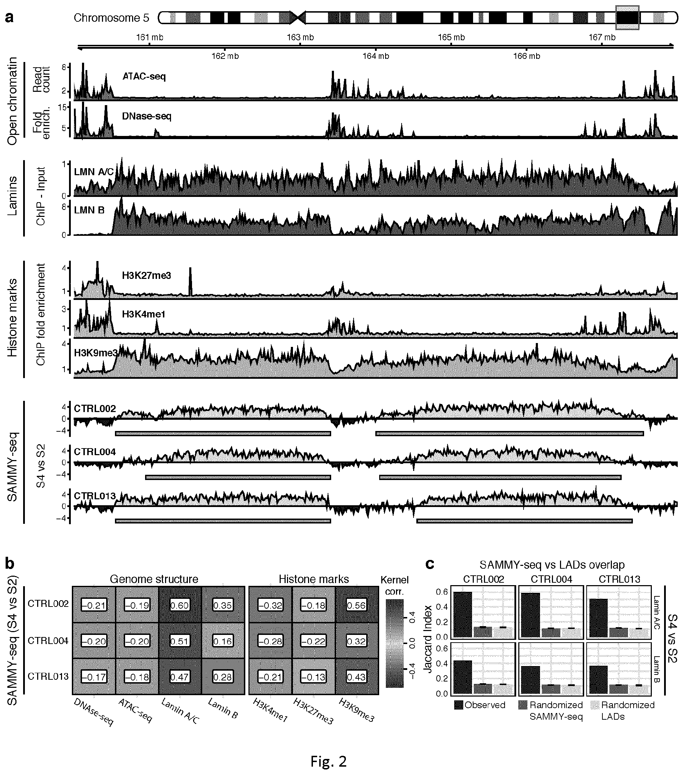

**Figure 2****. SAMMY-seq preferentially isolates heterochromatic domains. a,** Visualization of SAMMY-seq enrichment signal along with multiple chromatin marks on a representative region (8Mb region in chr5:160000000-168000000). From top to bottom: tracks for open chromatin (ATAC-seq and DNase-seq ― dark green); association to lamina (Lamin A/C and Lamin B ChIPseq ― dark blue); ChIP-seq for histone marks associated to PcG regulation (H3K27me3 ― light green), active chromatin (H3K4me1 ― light green) or heterochromatin (H3K9me3 ― light blue); SAMMY-seq enrichment signal (S4 vs S2) in 3 control samples (yellow or purple colored track for enrichment or depletion, respectively, over the S2 reference baseline). Grey boxes under each SAMMY-seq track show the SAMMY-seq domains. b, Genome-wide kernel correlation (StereoGene) for all control samples SAMMY-seq (S4 vs S2 enrichment) compared to ATAC-seq, DNase-seq, and ChIPseq (Lamin A/C, Lamin B, H3K27me3, H3K4me1 and H3K9me3). c, Overlap (Jaccard Index - JI) of control samples SAMMY-seq domains (S4 vs S2) with LADs (Lamin A/C or Lamin B ChIPseq enrichment domains). The observed JI (blue bar) is compared to mean JI across 10,000 randomizations of SAMMY-seq domains (pink bar) or LADs (yellow bar) positions along the genome. All Jaccard Indexes are significantly higher than the randomized values (all empirical p-values < 0.0001). The black rectangle over the randomized values shows the +/- 2 standard error range.

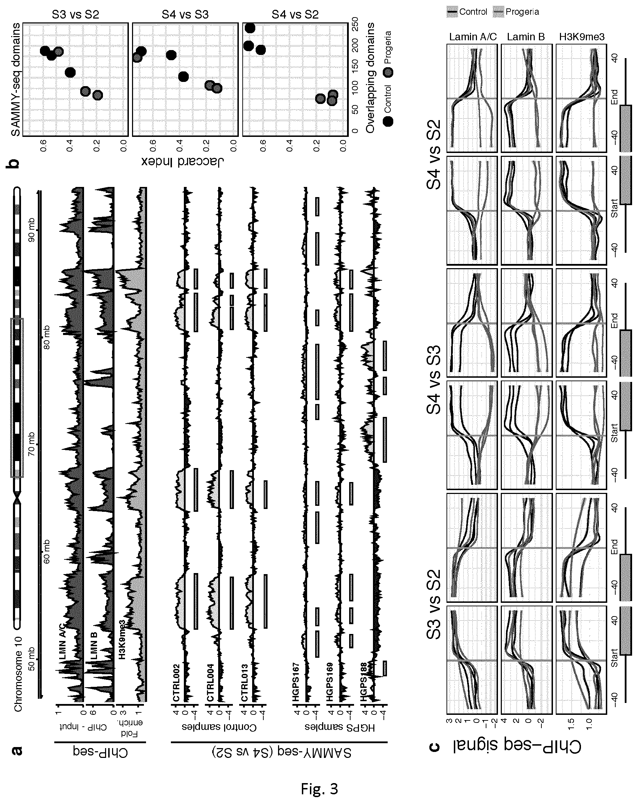

**Figure 3****. HGPS fibroblasts show early changes in chromatin accessibility. a,** Genomic tracks for SAMMY-seq in 3 control and 3 HGPS samples along with chromatin marks in a representative region (48 Mb region in chr10:46000000-94000000). From top to bottom: tracks for association to lamina (Lamin A/C and Lamin B ChIP-seq ― dark blue); heterochromatin (H3K9me3 ChIP-seq ― light blue); SAMMY-seq enrichment signal (S4 vs S2) in 3 control and 3 HGPS samples (yellow or purple colored track for enrichment or depletion, respectively, over the S2 reference baseline). Grey boxes under each SAMMY-seq track show the SAMMY-seq domains. b, Pairwise overlap of SAMMY-seq domains (S3 vs S2, S4 vs S3, S4 vs S2) between control (black dots) or HGPS (red dots) sample pairs (JI on y-axis, number of overlapping regions on x-axis). Upper-tail Fisher-test p-value < 0.01 for all overlaps, except for HGPS S4 vs S2 pairwise overlaps. c, Smoothed average ChIP-seq enrichment signal for chromatin marks around SAMMY-seq domain borders. Average ChIP-seq signal for Lamin A/C (upper row), Lamin B (middle row) and H3K9me3 (bottom row) is reported around the SAMMY-seq domain borders start (left side plots) or end (right side plots) for domains detected in each control (black lines) and progeria (red lines) sample. Results for each set of SAMMY-seq enrichment domains are reported (S3 vs S2 on the left, S4 vs S3 center, S4 vs S2 on the right) using a +/-50 bins window (10Kb bin size) centered on the start or end domain border positions (vertical dashed grey line).

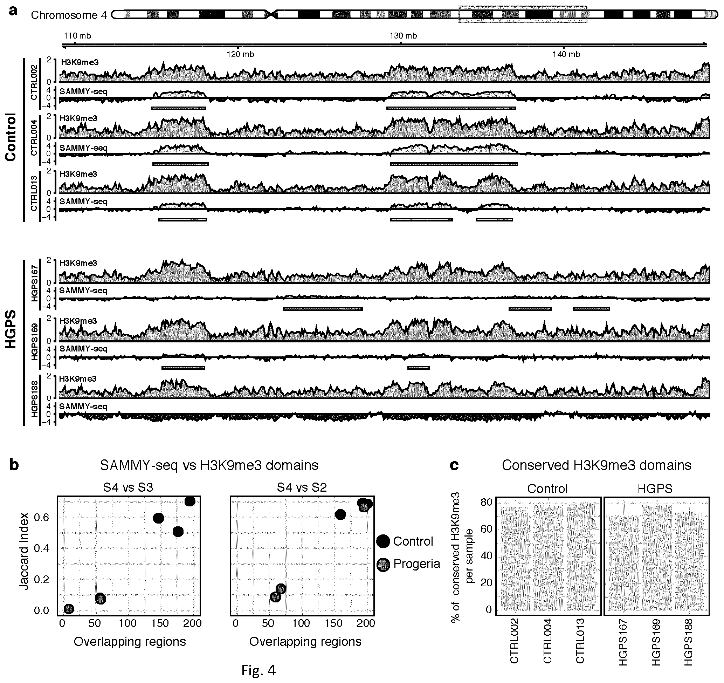

**Figure 4****. Early chromatin structure disruption in progeria is not accompanied by alterations in H3K9me3. a,** Genomic tracks for paired H3K9me3 ChIP-seq and SAMMY-seq in 3 control and 3 HGPS samples for a representative region (40Mb region in chr4:109000000-149000000). Enrichment signals (computed using SPP) for each sample are shown (H3K9me3 ChIP-seq ― light blue; yellow or purple colored track for SAMMY-seq S4 vs S2 enrichment or depletion, respectively). Grey boxes under each SAMMY-seq track show the SAMMY-seq domains. b, Overlap of H3K9me3 enriched domains and SAMMY-seq domains (S4 vs S3, S4 vs S2) for control (black dots) or HGPS (red dots) samples (JI on y-axis, number of overlapping regions on x-axis). All overlaps are significant in controls (for both S4 vs S3 and S4 vs S2 SAMMY-seq domains) (upper-tail Fisher-test p-value < 0.01 for all overlaps, except for HGPS S4 vs S2 pairwise overlaps), and also in HGPS169 (only for S4 vs S2 SAMMY-seq domains). c, Percentage of H3K9me3 domains conserved across all 3 control or all 3 progeria samples (computed as percent over per sample total H3K9me3 domains size).

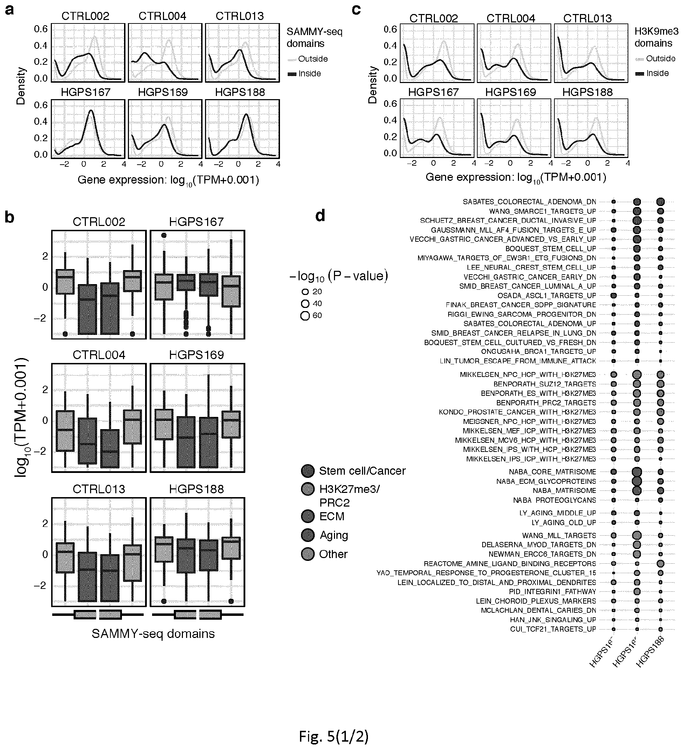

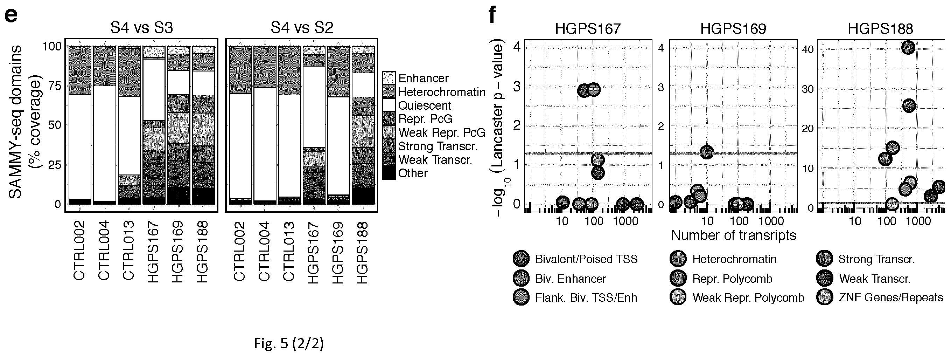

**Figure 5****. Chromatin structural changes affect PRC2 dependent transcriptional regulation. a,** Normalized gene expression distribution for protein coding genes separated based on their promoter being inside or outside of the SAMMY-seq domains (S4 vs S2) in each sample. Inventors considered only those genes with larger than 0 expression in at least one sample. Genes with promoters outside the domains have higher expression (Wilcoxon rank sum test pvalues < 0.01). **b**, Boxplot of normalized gene expression distribution for protein coding genes flanking SAMMY-seq domain borders (S4 vs S2) in each sample. The gray boxplots show genes located in the outer 500Kb flanking regions (upstream of start or downstream of end), the red boxplots show genes in the inner 500Kb flanking regions (all Wilcoxon rank sum test pvalues < 0.01, except for the HGPS167 domain ends). **c**, Gene expression distribution for protein coding genes separated based on their position being inside or outside of the H3K9me3 peaks in each sample. All samples show a significant difference in expression (Wilcoxon rank sum test p-values < 0.01). **d**, MSigDB pathways consistently deregulated in all three progeria samples (FDR corrected aggregated p-value < 0.05), based on comparison of individual HGPS samples to the group of control samples (transcript level expression). Pathways are ranked based on the sum of their -log₁₀ transformed p-values across all 3 progeria samples. Dot colors highlight different categories of pathways as per graphical legend: related to stem cells or cancer, H3K27me3/PRC2 regulation, extracellular matrix, aging or other categories. Dot size is proportional to the -log10 transformed p-value. **e**, Overlap of SAMMY-seq domains from each sample to Roadmap Epigenomics chromatin states for normal fibroblasts (E055). The overlaps with each chromatin state are reported as percentage over the total of SAMMY-seq domains (S4 vs S3 or S4 vs S2) for each sample. **f**, Transcripts differential expression (up-regulation) was assessed by comparing individual HGPS samples against the group of controls, then p-values were aggregated based on their chromatin state, considering only genes in SAMMY-seq domains (S4 vs S2). The plot reports the number of transcripts (x-axis) and significance (Lancaster method aggregated pvalues ― y-axis) for each chromatin state (color legend) as defined by³².

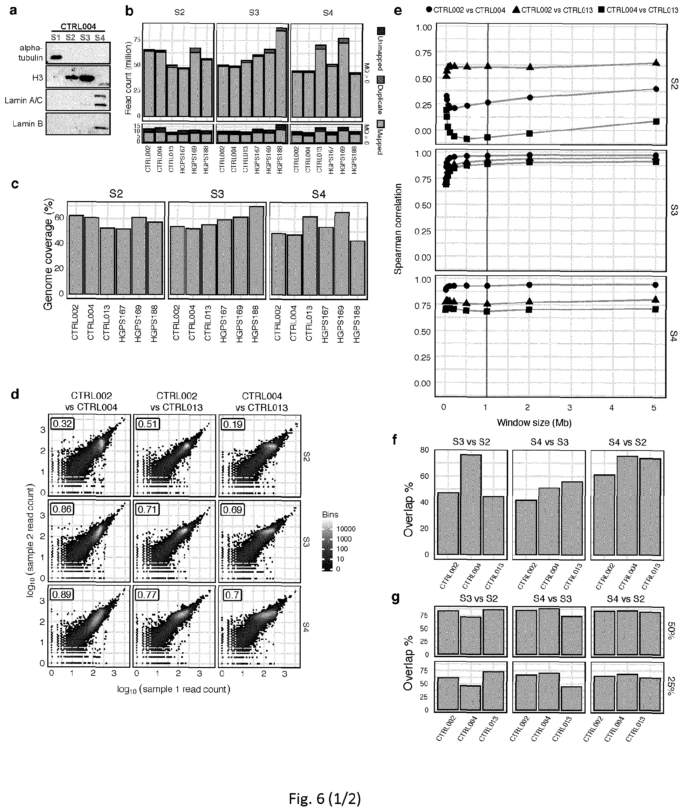

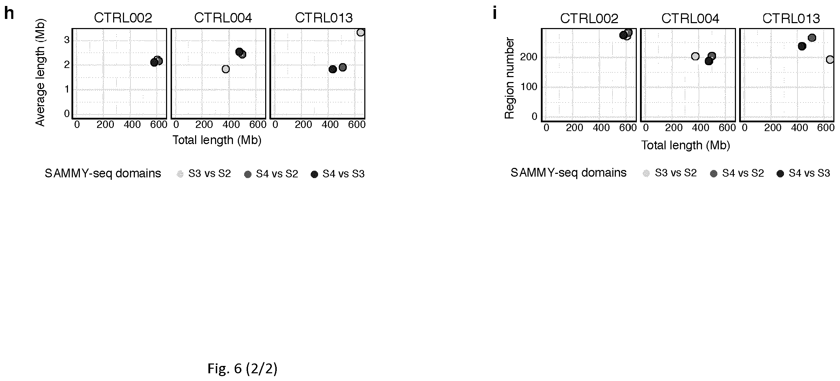

**Figure 6****. Characteristics and reliability of SAMMY-seq sequencing libraries. a,** Western blots for chromatin fractionation experiments of CTRL004. Sequential extractions were performed to isolate soluble proteins (S1-fraction), DNase-sensitive chromatin (S2-fraction), DNase-resistant chromatin (S3-fraction), and the most compact and inaccessible chromatin (S4-fraction). Equal amounts of each fraction were hybridized with indicated antibodies. **b**, Total read counts for each SAMMY-seq sample. The stacked bar plot shows mapped, unmapped, and PCR duplicate reads with bwa mapping quality equal to zero (MQ = 0, non-unique mapping) or larger than zero (MQ > 0, uniquely mapped reads). The average number of sequencing reads in the different fractions were 72 million (S2), 62 million (S3) and 65 (S4) million. **c**, Percent of the human genome (hg38-noalt build) covered by at least one read in each SAMMY-seq sample. On average 58% (S2), 54% (S3) and 52% (S4) of the genome was covered by at least one read. **d**, Scatter plots comparing read counts over 10Kb bins in the S2, S3 and S4 fractions (rows) across three pairwise comparisons of the control samples (columns). The color gradient represents the number of genomic bins with the same (x,y) values. Spearman correlation values are reported in the top left corner of each subpanel. **e**, Spearman correlation (y-axis) of read counts between control samples pairs (shape of dots) over different bin sizes (x-axis) in the S2, S3 and S4 fractions. The red line shows the 1Mb bin size. **f**, Percentage of SAMMY-seq domains (S3 vs S2, S4 vs S3 and S4 vs S2) conserved across all 3 control samples (computed as percent over per sample total SAMMY-seq domains size). **g**, Percentage of SAMMY-seq domains (S3 vs S2, S4 vs S3 and S4 vs S2) detected after 50% or 25% down sampling of sequencing reads (computed as percent over per sample total SAMMY-seq domains size detected with 100% of sequencing reads). **h**, Average (y-axis) and total (x-axis) size of SAMMY-seq domains (S3 vs S2, S4 vs S3 and S4 vs S2) for each control sample. Average size across samples is 2.47Mb for S3 vs S2, 2.16Mb for S4 vs S3 and 2.17Mb for S4 vs S2. Average genome coverage across samples is 18.84% S3 vs S2, 17.33% for S4 vs S3 and 18.85% for S4 vs S2. i, Number (y-axis) and total size (x-axis) of SAMMY-seq domains (S3 vs S2, S4 vs S3 and S4 vs S2) for each control sample.

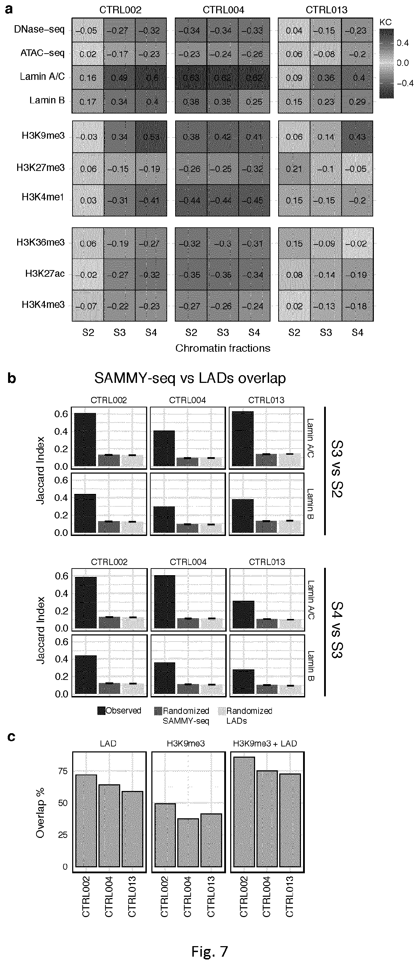

**Figure 7****. Detailed characteristics and genome-wide association of SAMMY-seq fractions. a,** Genome-wide kernel correlation calculated by StereoGene, between read coverage in individual control samples chromatin fractions against ChIP-seq and other enrichment signals in ATAC-seq, DNase-seq, Lamin A/C and Lamin B; H3K27me3, H3K4me1 and H3K9me3; H3K36me3, H3K27ac and H3K4me3. In most samples the correlation is progressively increasing for closed chromatin marks or decreasing for open chromatin marks when considering fractions from S2 to S4. b, Overlap (Jaccard Index - JI) of control samples SAMMY-seq domains (S3 vs S2 or S4 vs S3) with LADs (Lamin A/C or Lamin B ChIP-seq enrichment domains). The observed JI (blue bar) is compared to median JI across 10,000 randomizations of SAMMY-seq domains (pink bar) or LADs (yellow bar) positions along the genome. All Jaccard Indexes are significantly higher than the randomized values (all empirical p-values < 0.0001). The black rectangle over the randomized values shows the +/- 2 standard error range. **c**, Percentage of LAD, H3K9me3 or LAD+H3K9me3 regions detected by the S4 vs S2 SAMMY-seq domains. LADs were defined based on²⁷, while H3K9me3 regions were originating from the Roadmap Epigenomics E055 sample³².

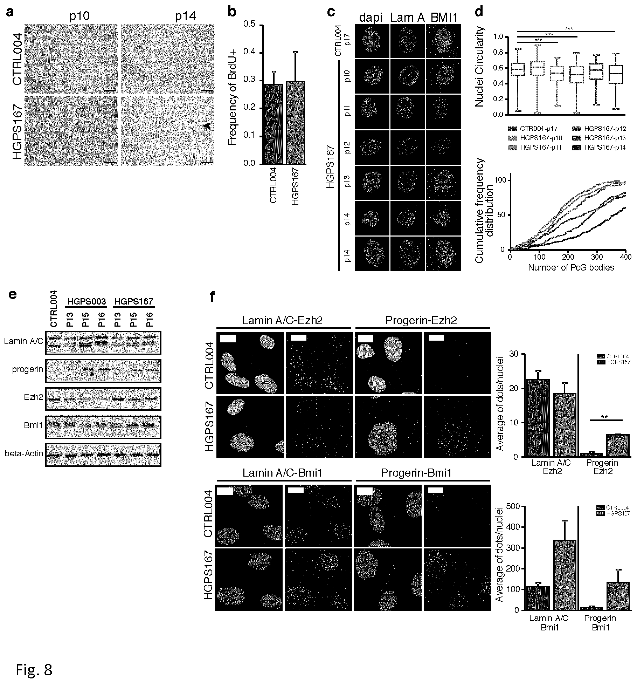

**Figure 8****. Characteristics of control and progeria fibroblast cells. a**, Representative fields of SA-b-gal activity in CTRL and HGPS fibroblasts at p10 and p14. Scale bar, 50 µm. **b**, Bar plot reporting the proliferation rate in control vs HGPS fibroblasts estimated as percentage of BrdU positive cells with respect to the total number of nuclei (n > 550). The bars report averages of two independent experiments, whiskers represent standard deviation (n = 2. **c,** Representative images of Bmi1 immunochemical analysis on fibroblasts from control (CTRL004) and HGPS167 at various passages. Nuclear lamina was stained with Lamin A antibody and nuclei with dapi. **d**, Quantification of nuclei circularity of CTRL004 and HGPS167 fibroblasts from p10 to p14 are represented above. Cumulative frequency distributions of the number of PcG bodies/nucleus for each cell populations are below. Comparisons by non-parametric Kruskal-Wallis test. **e**, Western blots of total protein extracts hybridized with the indicated antibodies in control and HGPS cells at different passages (p13, p15 and p16, as indicated). Beta-Actin was used as loading control. **f**, Representative images of PLA experiments in CTRL004 and HGPS167 at late passages (p18). Each fluorescent dot represents the co-localization of Lamin A/C or Progerin and Ezh2 (panel b) or Bmi1 (panel c). Nuclei were stained with dapi. All data were generated from an average of three independent experiments, whiskers represent SEM. Comparisons were done using a two-tailed t-test. Statistically significant differences are marked ^{∗ ∗} p < 0.01.

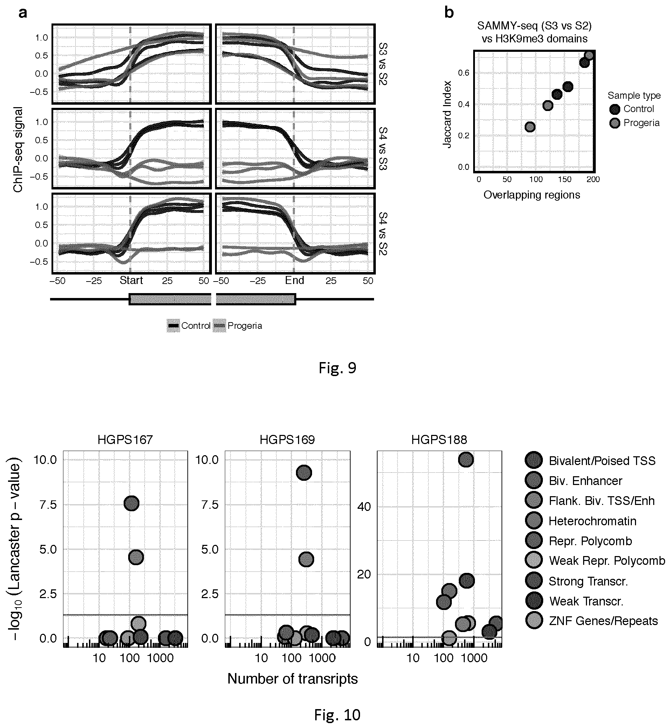

**Figure 9****. Additional analysis on H3K9me3 patterns. a**, Average ChIPseq enrichment signal for H3K9me3 is reported around the SAMMY-seq domain borders start (left side plots) or end (right side plots) for domains detected in each control (black lines) and progeria (red lines) sample. H3K9me3 ChIP-seq was obtained for each sample individually. Results for each set of SAMMY-seq enrichment domains are reported (S3 vs S2 top, S4 vs S3 middle, S4 vs S2 bottom) using a +/-50 bins window (10Kb bin size) centered on the start or end domain border positions (vertical dashed grey line). **b**, Overlap of H3K9me3 enriched domains and SAMMY-seq domains (S3 vs S2) for control (black dots) or HGPS (red dots) samples (JI on y-axis, number of overlapping regions on x-axis).
**Figure 10****.** Genome-wide association of enrichment domains and gene expression changes. Transcripts differential expression (up-regulation) was assessed by comparing individual HGPS samples against the group of controls, then p-values were aggregated based on their chromatin state, considering only genes in SAMMY-seq domains (S4 vs S3). The plot reports the number of transcripts (x-axis) and significance (Lancaster method aggregated p-values ― y-axis) for each chromatin state (color legend) as defined by Roadmap Epigenomics³².
**Figure 11****.** Visualization of S2L vs S3 SAMMY-seq enrichment signal along with chromatin marks on a representative genomic region (20Mb region in chr5:149642000-170000000). From top to bottom: SAMMY-seq enrichment signal (red or blue colored track for enrichment or depletion, respectively, over the S3 reference baseline), Roadmap E055 DNase-seq marking open chromatin (dark green); Roadmap E055 H3K9me3 marking heterochromatin (light blue).

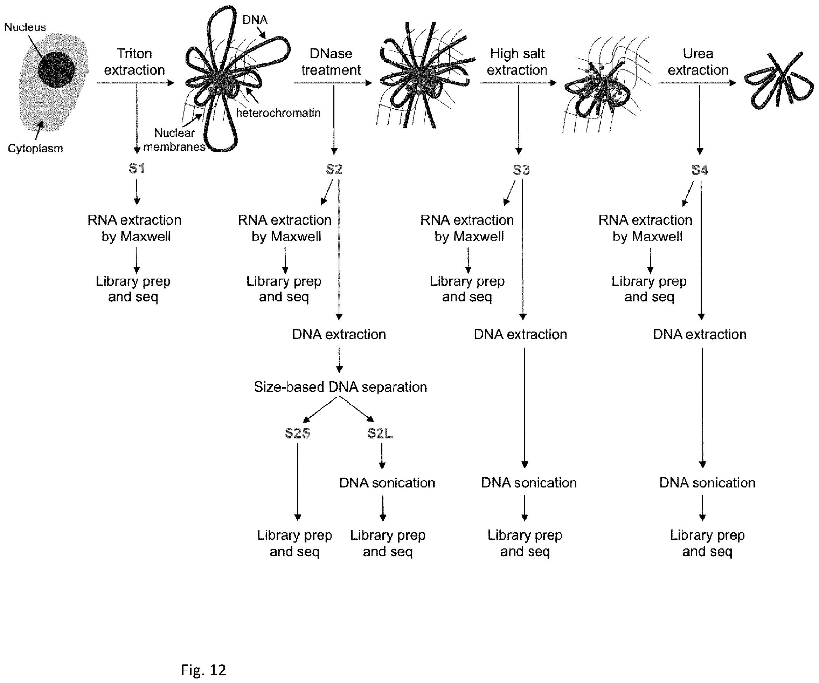

**Figure 12****.** Scheme of the method according to the invention performed on RNA or DNA.

### EXAMPLE 1

### Materials and Methods

### Cells or tissue

The below protocol was tested on cells (in particular human fibroblasts, lymphocytes and murine myoblasts) and tissues (such as human prostatic biopsies). The number of cells is variable between 0,5-20^{∗}10⁵ cells.

### Buffer components and Reagents

1. IX Phosphate-Buffered Saline (PBS) 2. 500 mM Pipes pH 6.8 3. 5 M NaCl 4. 1 M Sucrose 5. 1 M MgCl₂ 6. 500 mM EGTA 7. 10 % Triton X-100 (Note 1) 8. 8 M Urea 9. 25 X Complete EDTA-Free Protease Inhibitors Cocktail tablets (PIC) 10. 100 X Phenylmethanesulfonyl fluoride (PMSF) 11. 1 M Dithiothreitol (DTT) 12. RNAse free DNAse 13. 4 M (NH₄)₂SO₄ 14. 1 M Tris HCl pH 8 15. 500 mM EDTA 16. 10 mg / mL RNase A 17. 20 mg / mL Proteinase K 18. 25 24: 1 Phenol: Chlorophorm: Isoamilic Alcohol 19. 98% Ethanol 20. 3 M Sodium Acetate pH 5.2 21. Glycogen 22. 98% Ethanol 23. 10 mM Tris HCl pH 7.5 24. Dispase II 25. Collagenase A

### Equipment

1. Cell culture hood (i.e., biosafety cabinet) 2. Inverted microscope 3. Incubator set at 37°C, 5% CO₂ 4. Incubator set at 25°C 5. Micropipettes (1-10, 2-20, 20-200, 200-1000 µl) 6. Pipette aid 7. Burker camera 8. Refrigerated Centrifuge 9. Wheel at 4°C 10. Thermo-block 11. Freezers: -20°C and -80°C 12. Complete electrophoresis and western-blot apparatus 13. Oscillating shaker 14. Real Time qPCR machine 15. Centrifugal vacuum concentrator 16. Covaris M220 Focused-ultrasonicator 17. Agilent 2100 Bioanalyzer system

### Solutions

### Dispase/Collanegase buffer

2,4 Units/mL Dispase II; 2 µg/mL Collagenase A; 0,2 mM CaCl₂; 4 mM MgCl₂; 10 ng/mL Dnase I; PBS IX

### Citoskeleton (CSK) Triton buffer

10 mM Pipes pH 6.8; 100 mM NaCl; 1 mM EGTA; 300 mM Sucrose; 3 mM MgCl₂; 1 mM DTT; 0.5 % Triton X-100; 1 X PMSF; IX PIC; ddH₂O

Keep the buffer at 4°C just before use

### Citoskeleton (CSK) buffer

10 mM Pipes pH 6.8; 100 mM NaCl; 1 mM EGTA; 300 mM Sucrose; 3 mM MgCl₂; 1 X PMSF; IX PIC; dd H₂O

Keep the buffer at 4°C

### Citoskeleton (CSK) /NaCl buffer

10 mM Pipes pH 6.8; 2 M NaCl; 1 mM EGTA; 300 mM Sucrose; 3 mM MgCl₂; 1 X PMSF; IX PIC; ddH₂O

Keep the buffer at 4°C

### TE buffer

10 mM Tris HCl pH 8; 1 mM EDTA; ddH₂O

### SAMMY-seq procedure

1. Trypsinize cells (Note: To collect the cells avoid the use of scrapers in order to preserve cellular and nuclear structures and proteins compartmentalization. It is preferable to use cold PBS (stored at 4°C) to wash cells to prevent proteins degradation) (Note: Tissues: cut the tissue in small pieces and digest in a 2 mL of dispase/collagenase buffer for approximately 30-60 min until you have a cell suspension. Filter with 50 µm filter and collect cell suspension. Count cells as described in the step 3)
2. Inhibit trypsin resuspending the cells with media containing serum
3. Count the cells with Burker camera or preferred method (Note: For quantitative analysis and for comparison of distinct populations inventors suggest to count cells and to start from the same amount of cells.)
4. Centrifuge cells at 150 × g for 5 minutes at 4°C
5. Wash cells twice with cold physiologic solution, such as IX PBS, and centrifuge at 150 × g for 5 minutes at 4°C
6. Discard supernatant
7. Keep cell pellet on ice
8. Resuspend pellet in 600 µL of CSK Triton buffer (Note: For an optimal cellular permeabilization and proteins extraction inventors suggest to prepare a fresh 10% Triton X-100 solution; this in principle to avoid variability between experiments due to deterioration of the detergent. The solution can be stored in the dark at room temperature up to two weeks)
9. Syringe 10 times (Note: Use the syringe only for cells with abundant cytoplasm that are more difficultly lysed. For lymphocytes, syringe step is not needed.)
10. Keep on wheel for 10 minutes at 4°C
11. Centrifuge 3 minutes at 900 × g at 4°C
12. Collect supernatant designing it as S1 (Note: S1 fraction is enriched of the soluble nuclear and cytoplasmic proteins. Triton X-100 in a concentration between 0.1-1% is able to permeabilize plasma membrane and partially nuclear membrane, thus CSK Triton extraction allows the solubilization of both cytosolic and nuclear soluble proteins)
13. Resuspend the pellet in 600 µL of CSK Triton buffer (Note: Washes are essential to obtain uncontaminated fractions. In case of cross-contamination between fractions you can increase the number of washes and/or the volumes)
14. Keep on wheel for 10 minutes at 4°C
15. Centrifuge 3 minutes at 900 × g at 4°C
16. Discard the supernatant (Note: Remove gently the supernatant, keep attention in withdrawing all the supernatant using if needed p20 pipette to be more precise. This is required to obtain highly pure fractions)
17. Resuspend gently the pellet in 100 µL of CSK buffer
18. Add 25 U of RNAse ― free DNAse and incubate at 37°C for 1h
19. Add (NH₄)₂SO₄ to a final concentration of 250 mM, incubate 5 minutes on ice
20. Centrifuge 3 minutes at 900 × g at 4°C
21. Collect supernatant designing it as S2 (Note: S2 fraction is enriched in chromatin binding proteins. To avoid cross-contamination between S2 and S3 fractions, pay attention in carefully removing S2 phase avoiding contact of the viscous pellet (enriched in genomic DNA) with the microtips)
22. Resuspend the pellet with 200 µL of CSK buffer
23. Keep on wheel for 10 minutes at 4°C
24. Centrifuge 3 minutes at 3.000 × g at 4°C
25. Discard the supernatant
26. Resuspend gently the pellet in 100 µL of CSK NaCl buffer (Note: In CSK NaCl buffer, biological material tends to attach to the plastic tip. In order to preserve your sample and to have quantitatively reliable results, avoid extensive pipetting. Resuspend the pellet using a cut tip and perform subsequent washes just placing an appropriate amount of CSK NaCl buffer in the tube and flapping the tube several times to resuspend the pellet)
27. Keep on wheel for 10 minutes at 4°C
28. Centrifuge 3 minutes at 2300 × g at 4°C
29. Collect supernatant designing it as S3 (Note: The treatment in CSK NaCl buffer removes entirely the histones from the DNA. S3 fraction is enriched in histones and histone bound proteins)
30. Wash twice with 200 µL of CSK NaCl buffer
31. Keep on wheel for 10 minutes at 4°C
32. Centrifuge 3 minutes at 3.000 × g at 4°C
33. Resuspend the pellet in 100 µL 8 M Urea
34. Keep the tube at room temperature for 10 minutes, pipetting after 5 minutes
35. Collect supernatant designing it as S4 (Note: Urea treatment dissolves cellular membranes releasing membrane associated proteins. S4 fraction is enriched in nucleo-skeleton and membrane associated proteins and it is considered as nuclear matrix-containing fraction)
37. Store at ― 80°C. (Note: Inventors recommend to check the purity of the fractions running western blots with proper fraction-specific controls when setting up the technology).
38. Take an aliquot corresponding to 50% of S2 volume (about 55 µl) and to 100% of S3 and S4 volume (about 100µl) and bring to a final volume of 200 µl by adding TE buffer (Note: S1 fraction contains a negligible amount of DNA, thus is a good control to prove that the protocol have been performed correctly. It is recommended to extract DNA from 33% of S1 fraction as a negative control when setting up the technology)
39. Add 40µg of RNAse A and incubate at 37°C for 90 minutes
40. Add 40µg of Proteinase K and incubate at 55°C for 150 minutes
41. Add 200µl of 25:24:1 Phenol:Chloroform:Isoamilic Alcohol and vortex for 2 minutes
42. Centrifuge at 18000 × g for 8 minutes
43. Take the aqueous phase
44. Add 200 µl of TE buffer to the organic phase and vortex for 1 minute (Note: To improve the yield of the DNA extracted and for reproducibility between experiments, inventors strongly suggest to perform a back extraction adding equal volume of TE buffer to the phenol and repeating the extraction)
45. Centrifuge at 18000 × g for 8 minutes
46. Take aqueous phase and add it to the previous one
47. Add 400 µl of 25:24:1 Phenol:Chloroform:Isoamilic Alcohol to the aqueous phase and vortex for 2 minutes
48. Centrifuge at 18000 × g for 8 minutes
49. Take the acqueous phase
50. Add 20 µg of glycogen, 1/10 volume of NaAc 3M and briefly vortex the solution
51. Add 3 volumes of 100% Ethanol, briefly vortex and incubate in dry ice for 1 hour or at -20°C overnight
52. Centrifuge at 18000 × g for 30 minutes at 4°C
53. Wash DNA pellet with 500 µl of 70% ethanol
54. Centrifuge at 18000 × g for 15 minutes at 4°C
55. Remove supernatant and let the pellet air dry
56. Resuspend the pellet from S2 in 50 µl of sterile H2O, the pellets from S3, S4 (and S1) in 15-30 µl of sterile H2O
57. Incubate at 4°C overnight
58. Additionally purify the DNA of S2 fraction using PCR DNA Purification Kit (preferably QIAquick PCR Purification Kit); resuspend in a final water volume of 50 µL (Note: inventors use water heated at 65°C for the elution step)
59. If necessary, bring S3 and S4 to 15µl volume using a centrifugal vacuum concentrator at room temperature (starting from 30 µl, it will take approximately 10 minutes) and keep them at 4°C
60. Separate S2 into two subfractions using AMPure XP beads: mix the S2 fraction with magnetic beads using a ratio of 0,90/0,95 (Note: ratio is determined on the basis of the length of DNA fragments you wish to isolate)
61. After 1 minute at room temperature put the tube containing S2 fraction on the magnet
62. Collect the surnatant and keep it at room temperature. This fraction contains smaller DNA fragments, produced by DNAse treatment. (Note: This new fraction, the S2small (S2s) will be processed later (step 68)
63. Wash beads adding 180 µL of 85% Ethanol in the tube placed on the magnet
64. After 30 seconds, remove the ethanol
65. Resuspend the beads in 20 µL of water
66. After 1 minute at room temperature put the tube containing S2 fraction on the magnet
67. Collect the surnatant and keep it at room temperature. (Note: This fraction contains larger DNA fragments. This fraction, the S2larger (S2l) will be processed later (step 75))
68. Add magnetic beads to the S2s fraction following the formula: Sample volume^{∗}(1,8-0,90/0,95). (Note: starting from approximately 50 µL of initial S2, the beads volume will be approximately 42,50 µL)
69. After 1 minute at room temperature put the tube containing S2s fraction on the magnet
70. Discard the clear surnatant
71. Wash beads adding 180 µL of 85% Ethanol in the tube placed on the magnet
72. After 30 seconds, remove the ethanol
73. Resuspend the beads in 20 µL of water
74. After 1 minute at room temperature put the tube containing S2s fraction on the magnet
75. Collect the surnatant and keep it at room temperature.
76. Concentrate S2s and S2l subfractions to 15µl volume using a centrifugal vacuum concentrator at room temperature
77. Sonicate S2l, S3 and S4 fractions in a Covaris M220 Focused-ultrasonicator using screw cap microTUBEs. Water bath at 20°C to maintain constant the temperature, peak power 30.0, duty factor 20.0, cycle/burst 50, duration: 125 seconds for S2l and S3 fractions, 150 seconds for S4 fraction.
78. Quantify 1 µL of DNA using Qubit (Note: This measure is necessary to avoid DNA overloading on Bioanalyser. If DNA concentration is more than 50-70 ng/µL, it is recommended to run a dilution, preferably 4ng/run)
79. To check sonication run 1 µL on Bioanalyzer 2100 using Agilent High Sensitivity DNA Kit. DNA fragments should form a smear, peaking between 150-200 bp. (Note: Avoid this step when the starting material is low (starting samples between 3-5^{∗}10⁵ cells). However, inventors strongly suggest to set up the procedure in advance, by running on Bioanalyzer a complete experiment)
80. Quantify 1 µL of DNA using Qubit. (Note: inventors use DNA double strand high sensitivity kit)
81. Process the rest of the sample for DNA sequencing
82. Libraries are prepared from sonicated DNA. Single-ended 50 bp-long reads are produced to reach a sequencing depth of about 30 million reads using an Illumina machine.

The above protocol is used in case of DNA analysis. When the nucleic acid to be analyzed is RNA, the fractioning methods used are the same as described in the above protocol. However, the fractions are then directly extracted, preferably with a Promega Kit. Fractions are put in an RNA extraction buffer. In particular, after step 12. an RNA extraction is performed by putting 1/3 of S1 surnatant in buffer Maxwell (obtained by mixing the 1-thioglycerol and homogenization solution) (Kit AS 1460). The extracted RNA is then stored at -80°C. After step 21., 29. and 35, an RNA extraction is carried out by putting all S2, all S3 or all S4 surnatant respectively, in buffer Maxwell (obtained by mixing the 1-thioglycerol and homogenization solution) (Kit AS 1460). The RNAs are then stored at -80°C. The subfractionation and the sonication (from step 60. to step 77) are performed only after DNA extraction and not in case of RNA extraction. After the extraction, RNA is evaluated on Bioanalyser 2100 using Agilent RNA 6000 Nano or Pico Kit, libraries are prepared and the RNA is sequenced. Strand specific, Paired-ended 75 bp-long reads are produced to reach a sequencing depth of about 80 million reads using an Illumina machine.

### Results

### Optimization of SAMMY seq with 4 fractions.

Inventors sequenced DNA after subfractionation of S2 fraction (Fig. 11). Inventors found a good corrispondance between the S2L/S3 enrichment with the open chromatin highlighted by the DNAse-seq. On the other hand the same S2L/S3 regions are not enriched by the heterochromatin H3K9me3 mark.

### EXAMPLE 2

### Cell cultures

Primary fibroblast cell lines were cultured in DMEM High glucose with glutamax supplemented with 15% FBS and 1% Pen/Strep. HGADFN167 (HGPS167), HGADFN169 (HGPS169), HGADFN188 (HGPS188), human dermal fibroblasts derived from HGPS patients were provided by the Progeria Research Foundation (PRF). AG07095 (CTRL002) human dermal fibroblasts were obtained from the Coriell Institute. Preputial fibroblast strain #2294 (CTRL004) was a generous gift from the Laboratory of Molecular and Cell Biology, Istituto Dermopatico dell'Immacolata (IDI)-IRCCS, Rome, Italy", while control dermal fibroblast CTRL013 was kindly provided by the Italian Laminopathies Network.

### Histochemistry, immunofluorescence assay and PLA analysis

Beta-galactosidase assay was performed to assess cellular senescence according to⁵⁴. Briefly, cells were fixed at room temperature for 10min in paraformaldehyde at 4%, washed twice with 1×PBS, and incubated with fresh staining solution at 37 °C for 16 hrs in dark room. Then cells were washed twice with 1×PBS, overlaid with 70% glycerol/PBS, and photographed. For immunofluorescence assay, coverslips were fixed with paraformaldehyde at 4% in PBS for 10 minutes. Then, cells were permeabilized with 0,5% triton X-100 in PBS and non-specific signals were blocked with 1% BSA in PBS for 30 minutes at room temperature. Incubation with antibodies for Bmi1 (Millipore 05-637, mouse) diluted 1:100 and Lamin A/C (Santa Cruz sc-6215, goat) diluted 1:200 was performed respectively 12-16 hrs at 4°C or at room temperature for 2 hours. Primary antibodies were diluted in a PBS solution containing BSA 1%. Cells were stained with appropriate secondary antibodies, diluted 1:200 for 1 h at room temperature. Washes were done in PBT. As secondary antibodies inventors used Alexa Fluor 488 Donkey antimouse IgG (715-545-150) and Alexa Fluor 594 Donkey anti-goat IgG (705-585-003), from Jackson ImmunoResearch Laboratories. Finally, DNA was counterstained with DAPI, and glasses were mounted in Vectashield Antifade (Vector Laboratories) or ProLong Gold Antifade Reagent (Invitrogen). For BrdU (5-Bromo-2¢-deoxy-uridine, Sigma B9285) labelling, cells were grown for 8 hrs in the presence of 50microM of BrdU, and then fixed in 4% PFA. After Triton X- 100 treatment, cells were incubated for 2 min at RT in 0.07N NaOH, briefly rinsed twice in PBS and blocked in PBS/1% BSA. Reaction with BrdU antibody (1:10, Becton Dickinson 347580) was performed at room temperature for 1 hour in a PBS solution containing BSA 1%. For PLA experiments, coverslips were fixed first with paraformaldehyde at 4% in PBS for 10 minutes. Then, cells were permeabilized with 0,5% triton X-100 in PBS and blocked with 1% BSA in PBS for 1 hour at room temperature. Incubation with progerin (Alexis human mAb, 13A4, ALX-804-662-R200) diluted 1:20 or Lamin A/C (Santa Cruz sc-6215, goat) diluted 1:200 was performed for 12-16 hrs at 4°C. After PBT washes, cells were incubated with Ezh2 (Cell signaling 4905S, rabbit 1:100) or Bmi1 (Abcam ab85688 rabbit 1:100) for 12-16 hrs at 4°C. Primary antibodies were diluted in a PBS solution containing BSA 1%. Finally, detection of protein interactions was performed using the Duolink system (Sigma) following manufacturer's instructions.

### Image analysis

Fluorescent images were taken with a Nikon ECLIPSE 90i microscope (1006 objective), equipped with a digital camera (Nikon Coolpix 990) and NIS Element software or with confocal Leica SP5 supported by LAS-AF software (version 2.6.0). Confocal image size was 1024×1024. PLA blobs quantification was performed using Cell Profiler 2.0. In order to automatically detect and quantify PcG bodies in fluorescence cell images inventors developed an algorithm that implements a method derived from the one described in⁵⁵. The algorithm performs the segmentation of cell nuclei and the detection of PcG bodies of each image. It then measures the area of nuclei and the number and area of the PcG bodies. The algorithm has been implemented in MATLAB following this pseudocode:

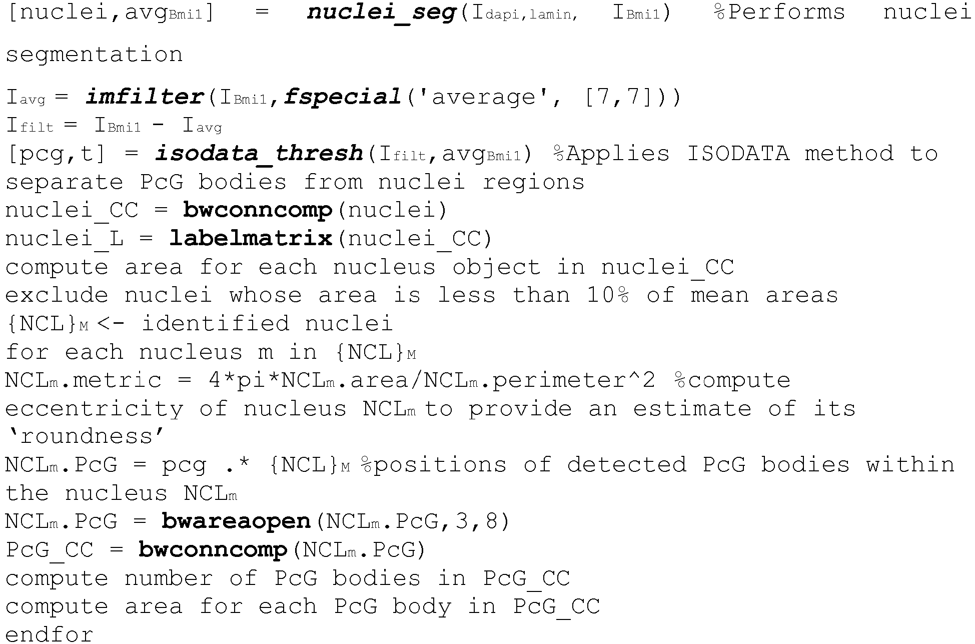

I_{dapi}, lamin is the image obtained by the sum of both images showing the fluorescence of nucleus and lamin. The function *nuclei_seg* performs a partition of cell image I_{dapi}, lamin in nuclei regions and background implementing a region based segmentation algorithm⁵⁶. nuclei is the binary image defining nuclei regions while avg_{Bmi1} is the mean intensity value of the nuclei regions in the image I_{Bmi1} that shows the fluorescence of PcG bodies. In order to better enhance PcG areas inventors subtract from the original image I_{Bmi1} its smoothed version obtained by applying an averaging filter of size 7, obtaining the image I_{filt}.

The function *isodata_thresh* implements the ISODATA classification algorithm⁵⁷ and uses relevant values computed by *nuclei_seg* function in order to extract PcG bodies from the nuclei regions. It sets the initial threshold value of ISODATA method as avg_{Bmi1}. pcg is the binary image defining PcG bodies. Reconstructions of nuclei are obtained through the connected components algorithm (*bwconncomp* MATLAB function, using a connectivity of 6). Nuclei are then labeled by applying the *labelmatrix* MATLAB function so they can be easily separated from each other. The algorithm computes the area of each reconstruction, discarding objects whose area is less than 10% of mean areas which are just noise. The algorithm uses the *bwareopen* function in order to discard too small detected PcG objects which are probably just noise. Reconstructions of PcG bodies are obtained through the connected components algorithm (*bwconncomp* MATLAB function, using a connectivity of 6). The algorithm computes the number of the PcG bodies and the area of any PcG body.

### Protein extraction and Western Blot analyses

Total proteins were prepared by resuspending 1×10⁶ cells in extraction buffer (50 mM TrisHCl pH 7.6; 0.15 M NaCl; 5 mM EDTA; 16 Protease Inhibitors; 1% Triton X-100). One 40sec pulse of sonication (UP100H manual sonicator, Hielscher) at 40% amplitude was performed to allow dissociation of protein from chromatin and solubilization. Extracts were analyzed by SDS-PAGE using an 8% gel (37.5:1 Acryl/Bis Acrylamide). The following primary antibodies were used: Beta-Actin (Santa-Cruz sc1616, rabbit 1:4000), Lamin A/C (Santa Cruz sc-6215, goat 1:4000), progerin (13A4 mouse, Abcam 66587, mouse 1:1000), Ezh2 (AC22 Cell Signaling 3147S, mouse 1:1000), Bmi1 (D42B3 Cell signaling, rabbit 1:1000). HRP-conjugated secondary antibodies were revealed with the ECL chemiluminescence kit (Thermo Fisher Scientific).

### Chromatin fractionation

Chromatin fractionation was carried out as described in²⁹ with minor adaptions. Briefly, 4 million fibroblasts were washed in PBS IX, and extracted in cytoskeleton buffer (CSK: 10 mM PIPES pH 6,8; 100 mM NaCl; 1 mM EGTA; 300 mM Sucrose; 3 mM MgCl₂; IX protease Inhibitors by Roche Diagnostics; 1 mM PMSF) supplemented with 1 mM DTT and 0,5% Triton X-100. After 5 min at 4°C the cytoskeletal structure was separated from soluble proteins by centrifugation at 3000 rpm for 3 min, and the supernatant was labeled as S1 fraction. The pellets were washed with an additional volume of cytoskeleton buffer. Chromatin was solubilized by DNA digestion with 25U of RNase―free DNase (Invitrogen) in CSK buffer for 60 min at 37°C. To stop digestion, ammonium sulphate was added in CSK buffer to a final concentration of 250 mM and, after 5 min at 4°C samples were pelleted at 5000 rpm for 3 min at 4°C and the supernatant was labeled as S2 fraction. After a wash in CSK buffer, the pellet was further extracted with 2M NaCl in CSK buffer for 5 min at 4°C, centrifuged at 5000 rpm 3 min at 4°C and the supernatant was labeled as S3 fraction. This treatment removed the majority of histones from chromatin. After 2 washing in NaCl 2M CSK, the pellets were solubilized in 8M urea buffer to remove any remaining protein component by applying highly denaturing conditions. This remaining fraction was labeled as S4. Supernatants from each extraction step were quantified and analyzed by SDS-PAGE and immunoblotting. Anti-tubulin alpha (Sigma T5168, mouse 1:10000), H3 (Abcam ab1791, rabbit 1:4000), Lamin A/C (Santa Cruz sc-6215, goat 1:4000) and Lamin B (Santa Cruz sc-6216, goat 1:2000) were used as primary antibodies. HRP-conjugated secondary antibodies were revealed with the ECL chemiluminescence kit (Thermo Fisher Scientific).

### DNA sonication and sequencing for chromatin fractionation

For DNA fractionation, inventors took an aliquot corresponding to 30% of volume of S2, S3, S4 fractions and added TE buffer to reach a final volume of 300µL. After incubation with RNAse A (Roche) (90 minutes at 37°) and Proteinase K (Sigma) (150 minutes at 55°), DNA was extracted from beads by standard phenol/chloroform extraction, precipitated and resuspended in 25µl milliQ H2O. After Nanodrop (260/280 = 1,7-1,9; 260/230 ≥ 2) and Qubit HS DNA quantification, inventors added H2O to a final volume of 105µL. Then samples were transferred to 96 well plates and sonicated 4 times with Bioruptor sonicator (10 minutes 30 seconds ON - 30 seconds OFF, High Power). The DNA profiles were finally checked by capillary electrophoresis (Agilent 2100 Bioanalyzer). Finally, DNA libraries were prepared by using NuGEN Ovation Ultralow Library Prep System kit and then sequenced using an Illumina HiSeq 2500 instrument according to manufacturer's instructions (Illumina).

### Chromatin immunoprecipitation and sequencing

Cells were cross-linked with 1% HCHO for 12 minutes at room temperature, lysed and chromatin sheared using Branson Digital Sonifier. IP were performed overnight on a wheel at 4° with 2.4 micrograms of H3K9me3 antibody (ab8898, Abcam). The following day, antibodychromatin immunocomplexes were loaded onto Dynal G magnetic beads (Invitrogen 10004D); the bound complexes were washed once in Low Salt Solution (0,1% SDS, 2mM EDTA, 1% Triton X-100, 20mM Tris pH 8, 150 mM NaCl), once in High Salt Solution (0,1% SDS, 2mM EDTA, 1% Triton X-100, 20mM Tris pH 8, 500 mM NaCl), once again in Low Salt Solution and once in Tris/EDTA 50 mM NaCl. Crosslinking was reversed at 65°C overnight in Elution Buffer (50 mM Tris pH 8, 10 mM EDTA, 1% SDS) and DNA extracted from beads by standard phenol/chloroform extraction, precipitated and resuspended in 30 µl of 10 mM Tris pH 8. ChIP efficiency was tested by qPCR reactions, performed in triplicate (precipitated DNA samples as well as input DNA) using QuantiTect SYBR Green master mix (Qiagen) on a StepOnePlus^{™} Real-Time PCR System (Applied Biosystems). Relative enrichment was calculated as ChIP / Input ratio. Libraries for sequencing were created using the automation instrument Biomek FX (Beckman Coulter), then qualitatively and quantitatively checked using Agilent High Sensitivity DNA Kit (Agilent Technologies, 5067-4627) on a Bioanalyzer 2100 (Agilent Technologies). Libraries with distinct adapter indexes were multiplexed and, after cluster generation on FlowCell, were sequenced for 50bp in the single read mode on a HiSeq 2000 sequencer at the IEO Genomic Unit in Milan.

### RNA sequencing

For high-throughput sequencing, cDNA libraries were prepared from total RNA, extracted with Trizol, by using Illumina TruSeq Stranded Total RNA Kit with Ribo-Zero GOLD. cDNA fragments of ~300 bp were purified from each library and were sequenced for 125bp, using an Illumina HiSeq 2500 instrument according to manufacturer's instructions (Illumina).

### SAMMY-seq sequencing read analysis

Sequencing reads were aligned to the hg38-noalt reference human genome available in the bcbio-nextgen pipeline, using bwa aln⁵⁸ (version 0.7.12) with options -n 2 -k 2 and saved the results in sam format with bwa samse. The sam files were converted to bam and name sorted with samtools⁵⁹ (version 1.3.1). Inventors marked PCR duplicates using the biobambam2 toolset⁶⁰ (version 2.0.54). Inventors discarded reads mapping to non-autosomal chromosomes, PCR duplicates, qcfail, multimapping and unmapped reads with samtools. Inventors converted the bam files to bedgraph using bedtools⁶¹ (version 2.25.0) and bedgraph to bigWig using UCSC's bedgraphToBigWig tool (version 4) for reads distribution visualization. Read coverage was normalized by the total sequencing library size, before converting bedgraph files to bigWig. Sample quality was assessed using fastqc (version 0.11.5) (http://www.bioinformatics.babraham.ac.uk/projects/fastqc). For the down sampling analysis, inventors sampled the raw fastq files to 50% and 25% using the seqtk sample command (https://github.com/lh3/seqtk) and ran the same pipeline, as above.

### ChIP-seq sequencing read analysis

Sequencing reads were aligned, filtered, converted and quality checked using the same tools as for the SAMMY-seq reads. Before sequence alignment inventors did an additional trimming step, using Trimmomatic⁶² (version 0.32) in single end mode. Inventors used the Enriched Domain Detector (EDD)³¹ tool to call H3K9me3 peaks, with the following options: --fdr 0.1 ― gap-penalty 10 ―binsize 100 ―write-log-ratios ―write-bin-scores and also excluding blacklisted genomic regions containing telomeric, centromeric, and certain heterochromatic regions⁶³. Inventors also changed the required_fraction_of_informative bins parameter to 0.98.

### Literature data processing

Inventors collected publicly available datasets from the following sources: ATAC-seq from³⁴, Lamin A/C ChIP-seq from²⁷, Lamin B ChIP-seq from³³. Sequencing reads were aligned, filtered and converted using the same tools as for the SAMMY-seq reads. Inventors calculated the genome wide ChIP-seq signal for Lamin A/C and Lamin B data, using the SPP package (version 1.15.4)⁶⁴. Inventors imported bam files into the R (version 3.3.1) statistical environment, and selected informative reads with the get.binding.characteristics and select.informative.tags functions, removed anomalous positions with extremely high number of reads using theremove.local.tag.anomalies function, and calculated the differential signal, smoothed by a Gaussian kernel, using the get.smoothed.tag.density function with the default bandwidth and tag.shift parameter. Inventors used the filtered read count as the genome-wide signal for the ATACseq sample. Additionally, inventors downloaded from Roadmap Epigenomics³² the genome-wide signal coverage tracks for the DNAse-seq, as well as histone marks ChIP-seq datasets (H3K9me3, H3K27me3, H3K4me1, H3K36me3, H3K27ac, H3K4me3) for the foreskin fibroblast sample E055. Inventors converted bigwig files to bedgraph with the bigWigToBedGraph tool, lifted over genomic coordinates from hg19 to hg38 with the liftOver tool and converted back bedgraph to bigwig with the bedGraphToBigWig tool using the UCSC toolkit⁶⁵ (version 4). Inventors calculated genome-wide correlations between SAMMY-seq samples and all of the public datasets listed and processed above using StereoGene³⁵ (version 1.73). StereoGene uses kernel correlation to calculate genome-wide correlation for spatially related but not completely overlapping features irrespective of their discrete or continuous nature. Inventors called lamina associated domains with the EDD tool. For the Lamin A/C dataset inventors used the following options: ―gap-penalty 25 ―bin-size 200 ―write-log-ratios ― write-bin-scores, and for the Lamin B dataset inventors used: ―gap-penalty 5 ―bin-size 100 ― write-log-ratios ―write-bin-scores. Inventors also changed the required fraction_of_informative_bins parameter to 0.98.

### SAMMY-seq domain calling, signal calculation, overlap analysis and visualization

Inventors performed relative comparisons of SAMMY-seq fractions within each sample using EDD, optimized to call very broad enrichment domains. EDD was originally designed for lamin ChIPseq data, comparing IP and input samples. As SAMMY-seq data also shows broad enrichment regions, inventors used EDD to select significantly enriched SAMMY-seq domains by comparing less accessible fractions to more accessible ones (S3 vs S2, S4 vs S3 and S4 vs S2 comparisons) in each sample, with the following options: --gap-penalty 25 --bin-size 50 --write-log-ratios --writebin- scores and also excluding blacklisted genomic regions containing telomeric, centromeric, and certain heterochromatic regions63. Inventors also changed the required_fraction_of_informative_bins parameter to 0.98. Inventors used the same set of parameters for the down sampled data, except changing ―bin-size to 100 for 50% down sampling or 200 for 25% down sampling. Additionally, inventors calculated the genome wide differential signal for all comparisons, using the SPP package (version 1.15.4)⁶⁴. Inventors imported bam files into the R (version 3.3.1) statistical environment, and selected informative reads with the get.binding.characteristics and select.informative.tags functions, removed anomalous positions with extremely high number of reads using the remove.local.tag.anomalies function, and calculated the differential signal, smoothed by a Gaussian kernel, using the get.smoothed.tag.density function with the default bandwidth parameter and tag.shift = 0. Inventors used bedtools⁶¹ (version 2.25.0) and the bedtools jaccard or bedtools fisher command to calculate Jaccard Index or Fisher-test p-values for the various overlap analyses. Inventors randomized SAMMY-seq domains and LADs for the SAMMY-seq ― LAD overlap analysis using the bedtools shuffle with -noOverlapping option and also excluding blacklisted genomic regions containing telomeric, centromeric, and certain heterochromatic regions⁶³ with the-exclude option. Inventors used the gViz⁶⁶ and karyoploter⁶⁷ Bioconductor packages to visualize SAMMY-seq read coverages, differential signals and domains. Inventors used ggplot2⁶⁸ for additional plotting, and the GAM method⁶⁹ from the mgcv package (version 1.8-12) for smoothing data while plotting SAMMY-seq border region signals.

### RNA-seq sequencing read analysis

Transcript and gene level quantification was done with Kallisto⁷⁰ (version 0.43.0) to estimate transcript level read counts and TPM (Transcripts Per Million) values. The GENCODE v27 annotation was used to build the transcriptome index. Kallisto was run with the --bias option to perform sequence based bias correction on each sample. Inventors used sleuth⁷¹ (version 0.29.0) to calculate gene or transcript level differential expression comparing all three controls against all three HGPS samples, where the linear model used in sleuth included the sample type (control or HGPS), sex and library id. Additionally, inventors analyzed transcript level differential expression, comparing the three controls with a single progeria sample, where the linear model used in sleuth included the sample type (control or HGPS) and sex. For both cases, inventors used sleuth's Wald test to calculate p and q-values. After differential expression, inventors grouped transcripts according to MSigDB pathways and aggregated transcript p-values using the Lancaster aggregation method from the aggregation R package (version 1.0.1) motivated by a recently described analysis pipeline in⁷². Lancaster p-values were corrected for multiple testing using the Benjamini-Hochberg method. Alternatively, inventors aggregated transcript p-values based on their Roadmap chromatin states and differential expression direction. Inventors used two different criteria: for the Tx, TxWk, ReprPC, ReprPCWk, Het, and ZNF/Rpts chromatin states, inventors filtered for transcripts overlapping at least 50% with the state.For the EnhBiv, TssBiv and BivFlnk regions inventors required that the 200nt region around th TSS region overlaps at least 50% with the state. Inventors aggregated p-values of up-regulated (sleuth b-value > 0) or down-regulated (sleuth b-value < 0) transcripts separately, according to their chromatin state overlap and additionally filtering for being in a specific (S4 vs S3 or S4 vs S2) enrichment region.

### Data availability

The datasets generated during the current study are available in the GEO repository with accession number PRJNA483177.

### Results

### SAMMY-seq allows mapping of lamina associated heterochromatic regions

Inventors developed the SAMMY-seq method for genome-wide mapping of chromatin fractions separated by accessibility. The method is based on the sequential extraction of distinct nuclear fractions containing: soluble proteins (S1 fraction), DNase-sensitive chromatin (S2 fraction), DNase-resistant chromatin (S3 fraction) and the most condensed and insoluble portion of chromatin (S4 fraction) (Fig. 1a and Fig. 6a). The chromatin fractionation protocol was originally proposed by He et al.²⁹ and previously adapted by the present group for quantitative PCR of heterochromatin and lamin associated regions^{18,30}. Here inventors revised the protocol to leverage high throughput DNA sequencing of the S2, S3 and S4 fractions, for genome-wide mapping of the distinct chromatin fractions. Inventors first applied SAMMY-seq to 3 independent normal skin fibroblast cell lines, originating from 3 different individuals (control samples). The average number of reads in the different fractions was 72 million (S2), 62 million (S3) and 65 (S4) million (Fig. 1b, Fig. 6b, Table 1), and on average 58% (S2), 54% (S3) and 52% (S4) of the genome was covered by at least one read (Fig. 6c). The genome-wide correlation of read coverage confirms a high reproducibility of signal for the S3 and S4 fractions, enriched for more compacted chromatin, whereas the S2 fraction is more variable across biological replicates (Fig. 6d). Inventors noticed that correlation computed over genomic bins of variable sizes, ranging from 10Kb to 5Mb, becomes more stable with larger bin sizes (Fig. 6e). At 1Mb resolution, the mean Spearman correlation is 0.27 for the S2 fraction, 0.92 for S3, and 0.79 for S4. Visual inspection of read coverage profiles shows megabase-scale "bumps" in S3 and, more prominently, in S4 fractions (Fig. 1b). Thus, to detect relative enrichment of specific genomic regions between chromatin fractions, inventors applied the Enriched Domain Detector (EDD)³¹ algorithm to the present data. EDD was specifically designed to identify broad regions of enrichment in high-throughput sequencing results. Inventors looked for differentially enriched genomic regions using pairwise comparisons of less accessible vs more accessible fractions used as baseline: S3 vs S2, S4 vs S3 and S4 vs S2 (Fig. 1c). Inventors found a well-defined set of regions (SAMMY-seq domains) consistently enriched in the less accessible fractions. These domains are conserved across samples, with the S4 vs S2 comparison being the most consistent, and showing an average of 70.18% conservation (Fig. 6f). Repeating the differential enrichment analysis with down sampling the fractions to 50% of sequencing reads leads to similar SAMMY-seq domains, with an average overlap of 80.64 % for S3 vs S2, 82.22 % for S4 vs S3 and 83.42 % for S4 vs S2 across samples (Fig 6g). Down sampling to 25% still leads to an average of 61.92 % overlapping domains across samples and comparisons. Inventors also noticed that the SAMMY-seq domains have properties similar to LADs in terms of mean size and coverage (Fig. 6h, i, Table 2)¹⁶, thus inventors further investigated their properties. To functionally characterize the SAMMY-seq enriched regions, inventors compared the genome-wide read coverage with reference chromatin marks profiles for the same cell type. Using data from the Roadmap Epigenomics consortium³² and other publications^{27,33,34} inventors noticed that SAMMYseq signal (S4 vs S2 enrichment) is highly consistent across biological replicates (CTRL002, CTRL004, CTRL013), it is inversely correlated with open chromatin marks (ATAC-seq and DNase-seq) and is positively correlated with Lamin A/C and B ChIP-seq signal (Fig. 2a). Inventors confirmed this is a consistent genome-wide pattern using StereoGene kernel correlation³⁵ for the unbiased comparison of different types of chromatin marks (Fig. 2b). Similarly, when considering histone modifications inventors observed a positive association with heterochromatin marks (H3K9me3) and inverse correlation with active chromatin (H3K4me1) as well as Polycomb-regulated chromatin (H3K27me3) (Fig. 2a-b). The latter observation is compatible with previous reports that H3K27me3 is located at the border of heterochromatic LAD regions^{11- 13}. However, while on a large scale SAMMY-seq domains are located in H3K9me3 regions devoid of both H3K27me3 and H3K4me1, thus showing an inverse correlation, locally H3K4me1 and H3K27me3 have different patterns. Indeed, a closer inspection of histone marks profiles show that H3K4me1 peaks are located at positions of relatively lower H3K27me3 enrichment, and vice versa (Fig. 2a). The genome-wide correlation patterns are also consistent when considering individual SAMMY-seq fractions (Fig. 7a), confirming that inventors are progressively enriching for more compact chromatin in the sequential extraction steps. To quantify the overlap between SAMMY-seq enrichment regions and LADs, inventors used Lamin ChIP-seq datasets (Lamin A/C and Lamin B)^{27,33} and compared them with SAMMY-seq domains (S4 vs S2, S4 vs S3, S3 vs S2). On average 79% of SAMMY-seq domains across different comparisons and samples overlap with Lamin A/C LADs (Table 3). All of the comparisons show a significant overlap (average Jaccard Index 0.56 for Lamin A/C in S4vs S2), as assessed by randomizing either LADs or SAMMY-seq domains (Fig. 2c and Fig.7b). Interestingly, the overlap with Lamin A/C associated domains is 21% higher than with Lamin B associated domains, suggesting that SAMMY-seq preferentially enriches for regions interacting with Lamin A/C. This is also consistent with the observed higher genome-wide correlation with Lamin A/C compared to Lamin B (Fig. 2b and Fig. 7a). As a further confirmation that SAMMY-seq specifically detects the most compact heterochromatin, such as the lamina associated heterochromatic regions, inventors observed that SAMMY-seq domains have an even higher overlap with regions defined as both LADs and H3K9me3 enriched (77.84% average overlap), rather than considering each of the two markers independently (64.9% and 42.69% average overlap, respectively) (Fig. 7c).

### Progeria fibroblasts exhibit early alterations of chromatin architecture.

Inventors decided to apply the present technology on early passage HGPS fibroblasts, a cellular model of early stage progeria, as a challenging test case with subtle variations in chromatin structure. Indeed, according to recent literature, primary HGPS fibroblasts entering senescence show alterations in DNA interactions with Lamin A/C and in the distribution of PcG regulated H3K27me327. However, at early stages of senescence Hi-C experiments for genome-wide mapping of 3D chromatin architecture failed to detect alterations in the pairwise contact frequency between genomic loci. These were evident only at later-passage cells, representing more advanced stages of senescence as they exhibit severe nuclear blebs and inhibited proliferation²⁷. Inventors confirmed that early-passage HGPS fibroblasts do not show features normally associated with senescence such as beta-galactosidase positive cells and reduction in the proliferation rate (Fig. 8a, b). However, early defects in nuclear morphology are detectable (Fig. 8c, d). Progerin accumulates in HGPS fibroblasts while passaging cells (Fig. 8e). In parallel, as previously shown²⁸, a decrease is observed in the total level of Ezh2, the enzymatic subunit of PRC2 (Fig. 8e). To confirm a PcGprogerin crosstalk inventors used the proximity ligation assay (PLA), which detects even transient interactions between proteins in close proximity (< 30nm) within the nucleus of intact cells³⁶. In particular, inventors found that both Lamin A and progerin interact with PcG proteins (Ezh2, Bmi1) in age-matched control or HGPS fibroblasts (Fig. 8f). This is in line with expectations based on inventors' previous report that Lamin A interacts with PcG proteins¹⁸. Thus inventors checked intranuclear architecture of PcG bodies in HGPS progression (Fig. 8c), by analyzing the PRC1 subunit Bmi1, having similar protein level and distribution compared to control in HGPS fibroblasts (Fig. 8e). Inventors found a decrease in the number of PcG bodies per cell already at early passages (Fig. 8d). Conversely, late passage HGPS fibroblasts form larger Bmi1 aggregates, not detectable in control cells, suggesting an aberrant, progerin-dependent aggregation of Bmi1 protein (Fig. 8c). Taken together, these data confirm that increased levels of progerin can alter PcG proteins nuclear compartmentalization already in HGPS early stages. This is in line with recent literature^{18,27} while at the same time suggests that chromatin structure changes should be detectable at this stage, despite the recent failure to do so by Hi-C²⁷.

### SAMMY-seq detects chromatin changes in early passage progeria fibroblasts.

Inventors used SAMMY-seq to investigate chromatin changes in early-passage (passage number from 10 to 12) skin fibroblasts from 3 independent HGPS patients (Fig. 6a, b). Compared with analysis showing a consistent overlap (S4 vs S2) across control replicates, in HGPS patients the SAMMY-seq domains appear scattered and with generally lower signal to noise ratio (Fig. 3a). This would be compatible with a general loss of chromatin organization, resulting in more random distribution of genomic regions among the different fractions. Pairwise comparisons confirm that SAMMY-seq domains are largely overlapping among control samples (average Jaccard Index 0.67) whereas this overlap is lost in progeria samples, most notably in the S4 vs S2 comparison (average Jaccard Index 0.11) (Fig. 3b and Table 4). Moreover, SAMMY-seq enrichment in control samples is matching lamins and H3K9me3 ChIPseq enrichment, whereas this association is lost in HGPS SAMMY-seq samples (Fig. 3a). Inventors confirmed this is a genome-wide pattern by analyzing chromatin marks at SAMMY-seq domain borders (Fig. 3c). SAMMY-seq domains from control samples (S4 vs S2, S4 vs S3, S3 vs S2) correspond to regions normally enriched in Lamin A/C, Lamin B and H3K9me3 ChIP-seq reads, as opposed to flanking regions outside of domain borders. Progeria SAMMY-seq domains detected by comparing the intermediate and less compacted fractions (S3 vs S2) are similar to controls. However, when considering more compacted S4 fraction, the association to reference LADs and heterochromatin marks is lost in 2 patients for the S4 vs S2 and 3 patients for the S4 vs S3 comparisons, respectively (Fig. 3c). Here, both the lamins and the H3K9me3 signals are flattened out suggesting no specific association.

### Early changes of chromatin structure in HGPS fibroblasts is not accompanied by alterations of H3K9me3 patterns.

To further investigate how the remodeling of chromatin accessibility in early-passage HGPS fibroblasts correlates with changes in histone marks, inventors performed H3K9me3 ChIP-seq on the same set of control and HGPS fibroblasts analyzed by SAMMY-seq (Fig. 4a). Visual exploration of sequencing reads distribution confirms the consistent correlation between H3K9me3 ChIPseq and SAMMY-seq profiles for all of the control fibroblasts, whereas this association is lost in progeria samples (Fig. 4a). This is also confirmed by analyzing H3K9me3 genome-wide patterns at SAMMY-seq domain borders (Fig. 9a), where only controls show a correlation between SAMMY-seq and H3K9me3. In line with this findings, by using the EDD algorithm inventors identified broad H3K9me3 enrichment regions (average size 3.11Mb) which showed a large overlap with SAMMY-seq domains in control (average Jaccard Index of 0.67 for S4 vs S2 and 0.6 for S4 vs S3) and a small overlap in HGPS cells (average Jaccard Index of 0.3 for S4 vs S2 and 0.05 for S4 vs S3) (Fig 4b and Fig. 9b).

Interestingly, H3K9me3 ChIP-seq profiles are largely conserved in early-passage HGPS cells as compared to controls (Fig. 4a). This is confirmed by the genome-wide overlap of H3K9me3 enriched regions, which are largely conserved (between 69% and 80%) across control and HGPS samples alike (Fig. 4c).

### Large-scale chromatin structural changes affect expression of PcG regulated genes

Using RNA-seq on the same set of control and HGPS fibroblasts inventors assessed expression at the gene or transcript level detecting respectively 260 differentially expressed genes and 256 transcripts, suggesting that early stages of senescence in HGPS cells do not cause large scale changes in the transcriptome. To clarify if chromatin structural changes detected with SAMMYseq have an effect on transcription, inventors examined the expression of protein coding genes located in SAMMY-seq domains. As expected¹⁶, in all control samples genes within SAMMYseq domains (S4 vs S2) have lower expression levels than those outside (Fig. 5a). More specifically, in the genomic regions flanking SAMMY-seq domain borders inventors notice a clear pattern with a transition from lower expression within the domain to higher expression outside in all of the control samples (Fig. 5b). The same pattern is less marked or not present in HGPS samples (Fig. 5a,b). Inventors also verified that the transcriptional activity within H3K9me3 domains of each sample is repressed as expected (Fig. 5c), confirming that the inactive state of the regions is unchanged in HGPS.

As inventors noticed HGPS samples have unique SAMMY-seq domains, inventors compared individual HGPS expression profiles against the group of three controls to account for sample specific patterns. To verify which biological functions are affected inventors performed a pathway enrichment analysis using the MSigDB database³⁷ and selected the pathways commonly deregulated in all HGPS samples (Fig. 5d). Many of them are related to stem cells and cancer, in line with the hypothesis that accelerated aging affect stem cell niches³⁸. Interestingly, inventors also found several pathways related to PcG regulation (H3K27me3 and PRC2) (Fig. 5d). To further dissect the role of chromatin regulators in the disrupted expression patterns, inventors examined the overlap of SAMMY-seq domains with the chromatin states of normal skin fibroblasts³². In control samples, SAMMY-seq domains are almost entirely overlapping "Quiescent" or "Heterochromatin" states, as defined by Roadmap Epigenomics consortium³², whereas in HGPS cells SAMMY-seq domains comprise regions normally transcribed or regulated by PcG proteins (Fig. 5e). To understand the correlation between gene expression, chromatin states and altered structure, inventors grouped SAMMY-seq domain genes on the basis of their chromatin state in normal fibroblasts and examined their expression difference between normal and HGPS cells. This analysis revealed that Polycomb repressed and bivalent chromatin states are significantly differentially expressed between progeria and controls (Fig. 5f and Fig. 10). These findings overall suggest an involvement of PcG-related mechanisms in the disruption of gene expression regulation associated to early chromatin changes in HGPS.

### Discussion

Within the eukaryotic cell nucleus, chromatin acquires a specific structure which is fundamental for the correct spatiotemporal regulation of gene expression³⁹. In particular, heterochromatin is characterized by highly compact chromatin and specific nuclear compartmentalization. Important heterochromatin organizing regions include LADs, along with pericentric and telomeric regions, which are crucial for preserving chromosome structure^{3,4,10}. Alterations in heterochromatin structure and function are associated with developmental defects and cancer, while proper heterochromatin conformation is a hallmark of healthy cells^{40,41}. As such, reliable methods to characterize heterochromatin and its alterations are a crucial need for the biomedical scientific community. The development and adoption of new genome-wide experimental techniques based on nextgeneration sequencing (NGS) have been pivotal for advancing knowledge on chromatin structure and function⁴². Many of these widely adopted techniques, including ChIP-seq⁴³, ATACseq⁴⁴, DNase-seq⁴⁵ and MNase-seq⁴⁶, were originally designed to map active chromatin regions, whereas few options are available for mapping inactive heterochromatin regions or LADs. Here inventors present a new technology for mapping lamina associated heterochromatic regions called SAMMY-seq (Fig. 1). The protocol is based on the sequential extraction of multiple chromatin fractions, corresponding to increasingly compacted and less accessible chromatin regions, which are mapped along the genome using high-throughput sequencing. SAMMY-seq allows mapping non-accessible heterochromatin regions by the relative comparison of sequencing reads from different fractions (Fig. 2). Inventors show that SAMMY-seq can reproducibly and reliably identify lamina associated heterochromatic regions as the less accessible portions of chromatin in control wild type fibroblasts. On early passage HGPS fibroblasts (Fig. 3) inventors were able to detect chromatin structure alterations, in terms of accessibility changes, as measured by SAMMY-seq. Early alterations were postulated based on previous literature observations, also confirmed by inventors' own experimental data (Fig. 8). Nevertheless, previous Hi-C based data failed to detect early stage structural changes ²⁷. In line with previous findings showing that chromatin compartmentalization and histone marks deposition are independent processes^{47,48}, inventors also found that changes in chromatin accessibility are not accompanied by H3K9me3 alterations (Fig. 4 and ⁴⁹), further indicating that chromatin remodeling detected by SAMMY-seq is an early event in HGPS progression. On the other hand, detailed expression analysis highlighted the deregulation of a subset of PcG targets at this stage, including bivalent genes (Fig. 5). The present findings are in line with and reinforce previous studies showing that a correct chromatin architecture is necessary to cell fate determination^{50,51} and that alterations of PcG dependent H3K27me3 epigenetic mark occur in HGPS-derived cells^{27,28}. SAMMY-seq represents a significant improvement in the field of chromatin characterization as it provides novel information complementary and not overlapping with other high-throughput based sequencing methods, commonly used to study chromatin structure and function. These include ChIP-seq for mapping chromatin marks, DamID-seq for mapping association to nuclear lamina, and ATAC-seq or DNAse-seq for mapping chromatin accessibility. Of note, the present protocol is overcoming several major limitations of other methods for mapping lamina associated heterochromatic regions. First of all, the procedure can be applied on primary cells, as it doesn't require exogenous genes expression as DamID-seq. Then, SAMMY-seq does not involve chemical modifications of chromatin, which might cause artifacts and high background after sequencing^{52,53}. Moreover, it does not rely on antibodies for enriching specific chromatin fractions, thus avoiding issues related to antibody specificity, production, lot variability and cross-reactivity. This is particularly important when studying chromatin changes in cells where protein levels of chromatin associated factors could be altered, thus allowing more flexibility in terms of experimental design compared to antibody-based techniques. Globally the present results confirm SAMMY-seq is a powerful and flexible technique for mapping inactive chromatin regions characterized by more compacted and less accessible structure in primary cells. It is also sensitive to variations of chromatin structure, providing information complementary to other epigenomics experimental methods. The SAMMY-seq applied on primary cells allows to study heterochromatin structure alterations in physiological and pathological conditions such as laminopathies, aging and cancer.

### Tables

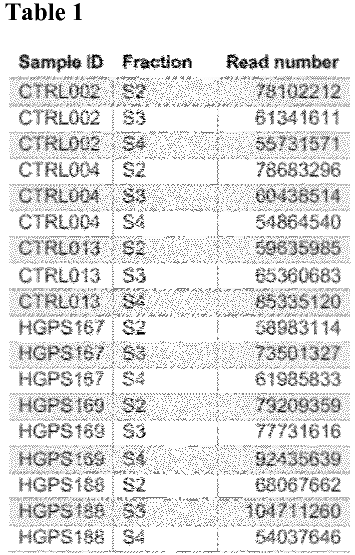

Total number of sequencing reads in all samples and fractions for the SAMMY-seq sequencing runs.

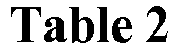

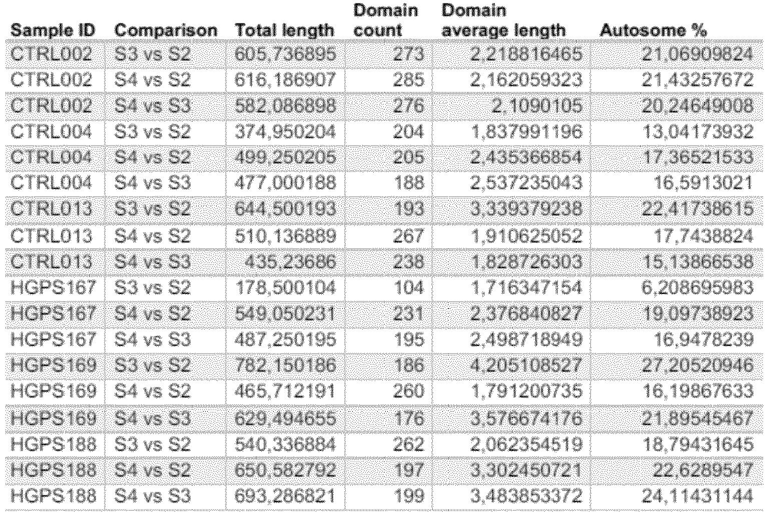

Characteristics of SAMMY-seq domains, detected by EDD. The table contains the following columns. Sample ID: patient ID, Comparison: fractions used in the EDD run as the "IP" and "input" sample, Total length: total length of SAMMY-seq domains in megabase, Domain count: number of SAMMY-seq domains, Domain average length: average length of SAMMY-seq domains in megabase, Autosome %: % of autosomes covered by SAMMY-seq domains.

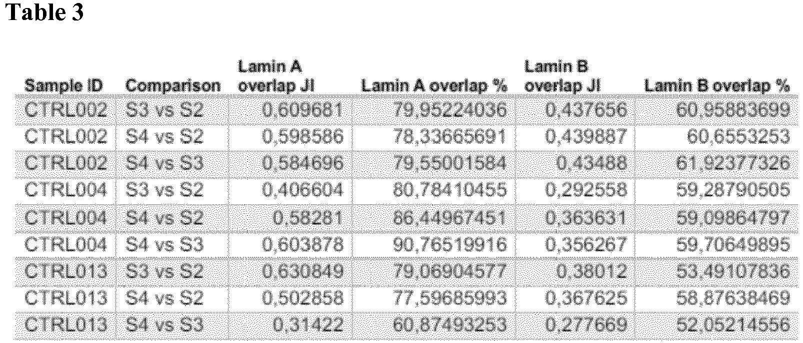

Statistics on control sample SAMMY-seq domains overlapping Lamina Associated Domains (LADs) based on literature data. The table contains the following columns. *Sample ID:* patient ID, *Comparison:* fractions used in the EDD run as the"IP" and "input" sample, *Lamin A overlap JI*: Jaccard index of the SAMMY-seq domain ― Lamin A/C LAD comparison as calculated by bedtools, *Lamin A overlap* %: % of SAMMYseq domains overlapping with Lamin A/C LADs, *Lamin B overlap JI*: Jaccard index of the SAMMY-seq domain ― Lamin B LAD comparison as calculated by bedtools, *Lamin B overlap* %: % of SAMMY-seq domains overlapping with Lamin B LADs.

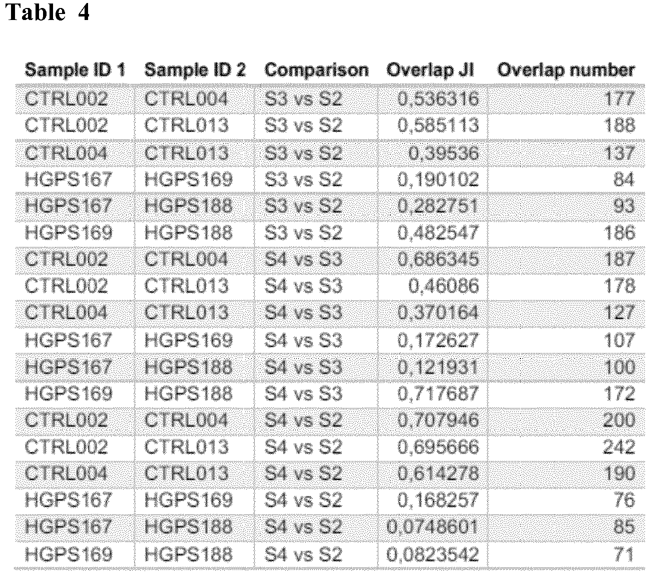

Pairwise comparison of SAMMY-seq domains across control or progeria samples. The table contains the following columns. *Sample ID 1:* patient ID for comparison, *Sample ID* 2: patient ID for comparison, *Comparison:* fractions used in the EDD run as the "IP" and "input" sample, *Overlap JI:* Jaccard index of the SAMMY-seq domain overlaps as calculated by bedtools, *Overlap number*: number of overlapping SAMMY-seq domains.

### References

1. Gilbert, N. et al. Chromatin architecture of the human genome: gene-rich domains are enriched in open chromatin fibers. Cell 118, 555-66 (2004).
2. Huisinga, K.L., Brower-Toland, B. & Elgin, S.C. The contradictory definitions of heterochromatin: transcription and silencing. Chromosoma 115, 110-22 (2006).
3. Blasco, M.A. The epigenetic regulation of mammalian telomeres. Nat Rev Genet 8, 299-309 (2007).
4. Gilbert, N. & Allan, J. Distinctive higher-order chromatin structure at mammalian centromeres. Proc Natl Acad Sci U S A 98, 11949-54 (2001).
5. Slotkin, R.K. & Martienssen, R. Transposable elements and the epigenetic regulation of the genome. Nat Rev Genet 8, 272-85 (2007).
6. Boettiger, A.N. et al. Super-resolution imaging reveals distinct chromatin folding for different epigenetic states. Nature (2016).
7. Schuettengruber, B., Bourbon, H.M., Di Croce, L. & Cavalli, G. Genome Regulation by Polycomb and Trithorax: 70 Years and Counting. Cell 171, 34-57 (2017).
8. Turgay, Y. et al. The molecular architecture of lamins in somatic cells. Nature 543, 261-264 (2017).
9. Shimi, T. et al. Structural organization of nuclear lamins A, C, B1, and B2 revealed by superresolution microscopy. Mol Biol Cell 26, 4075-86 (2015).
10. van Steensel, B. & Belmont, A.S. Lamina-Associated Domains: Links with Chromosome Architecture, Heterochromatin, and Gene Repression. Cell 169, 780-791 (2017).
11. Kind, J. et al. Single-cell dynamics of genome-nuclear lamina interactions. Cell 153, 178-92 (2013).
12. Kind, J. & van Steensel, B. Genome-nuclear lamina interactions and gene regulation. Curr Opin Cell Biol 22, 320-5 (2010).
13. Lund, E. et al. Lamin A/C-promoter interactions specify chromatin state-dependent transcription outcomes. Genome Res (2013).
14. Collas, P., Lund, E.G. & Oldenburg, A.R. Closing the (nuclear) envelope on the genome: How nuclear lamins interact with promoters and modulate gene expression. Bioessays 36, 75-83 (2014).
15. Meuleman, W. et al. Constitutive nuclear lamina-genome interactions are highly conserved and associated with A/T-rich sequence. Genome Res 23, 270-80 (2013).
16. Guelen, L. et al. Domain organization of human chromosomes revealed by mapping of nuclear lamina interactions. Nature 453, 948-51 (2008).
17. Briand, N. & Collas, P. Laminopathy-causing lamin A mutations reconfigure lamina-associated domains and local spatial chromatin conformation. Nucleus 9, 216-226 (2018).
18. Cesarini, E. et al. Lamin A/C sustains PcG protein architecture, maintaining transcriptional repression at target genes. J Cell Biol 211, 533-51 (2015).
19. Marullo, F. et al. Nucleoplasmic Lamin A/C and Polycomb group of proteins: an evolutionarily conserved interplay. Nucleus, 0 (2016).
20. Mattout, A. et al. An EDMD mutation in C. elegans lamin blocks muscle-specific gene relocation and compromises muscle integrity. Curr Biol 21, 1603-14 (2011).
21. Peric-Hupkes, D. et al. Molecular maps of the reorganization of genome-nuclear lamina interactions during differentiation. Mol Cell 38, 603-13 (2010).
22. Oldenburg, A. et al. A lipodystrophy-causing lamin A mutant alters conformation and epigenetic regulation of the anti-adipogenic MIR335 locus. J Cell Biol 216, 2731-2743 (2017).
23. Perovanovic, J. et al. Laminopathies disrupt epigenomic developmental programs and cell fate. Sci Transl Med 8, 335ra58 (2016).
24. Dirks, R.A., Stunnenberg, H.G. & Marks, H. Genome-wide epigenomic profiling for biomarker discovery. Clin Epigenetics 8, 122 (2016).
25. Ullrich, N.J. & Gordon, L.B. Hutchinson-Gilford progeria syndrome. Handb Clin Neurol 132, 249-64 (2015).
26. Vidak, S. & Foisner, R. Molecular insights into the premature aging disease progeria. Histochem Cell Biol 145, 401-17 (2016).
27. McCord, R.P. et al. Correlated alterations in genome organization, histone methylation, and DNAlamin A/C interactions in Hutchinson-Gilford progeria syndrome. Genome Res 23, 260-9 (2013).
28. Shumaker, D.K. et al. Mutant nuclear lamin A leads to progressive alterations of epigenetic control in premature aging. Proc Natl Acad Sci USA 103, 8703-8 (2006).
29. He, D.C., Nickerson, J.A. & Penman, S. Core filaments of the nuclear matrix. J Cell Biol 110, 569-80 (1990).
30. Marasca, F., Marullo, F. & Lanzuolo, C. Determination of Polycomb Group of Protein Compartmentalization Through Chromatin Fractionation Procedure. Methods Mol Biol 1480, 167- 80 (2016).
31. Lund, E., Oldenburg, A.R. & Collas, P. Enriched domain detector: a program for detection of wide genomic enrichment domains robust against local variations. Nucleic Acids Res 42, e92 (2014).
32. Roadmap Epigenomics, C. et al. Integrative analysis of 111 reference human epigenomes. Nature 518, 317-30 (2015).
33. Sadaie, M. et al. Redistribution of the Lamin B1 genomic binding profile affects rearrangement of heterochromatic domains and SAHF formation during senescence. Genes Dev 27, 1800-8 (2013).
34. Smith, D.K., Yang, J., Liu, M.L. & Zhang, C.L. Small Molecules Modulate Chromatin Accessibility to Promote NEUROG2-Mediated Fibroblast-to-Neuron Reprogramming. Stem Cell Reports 7, 955-969 (2016).
35. Stavrovskaya, E.D. et al. StereoGene: rapid estimation of genome-wide correlation of continuous or interval feature data. Bioinformatics 33, 3158-3165 (2017).
36. Fredriksson, S. et al. Protein detection using proximity-dependent DNA ligation assays. Nat Biotechnol 20, 473-7 (2002).
37. Liberzon, A. et al. The Molecular Signatures Database (MSigDB) hallmark gene set collection. Cell Syst 1, 417-425 (2015).
38. Scaffidi, P. & Misteli, T. Lamin A-dependent misregulation of adult stem cells associated with accelerated ageing. Nat Cell Biol 10, 452-9 (2008).
39. Sewitz, S.A., Fahmi, Z. & Lipkow, K. Higher order assembly: folding the chromosome. Curr Opin Struct Biol 42, 162-168 (2017).
40. Criscione, S.W., Teo, Y.V. & Neretti, N. The Chromatin Landscape of Cellular Senescence. Trends Genet 32, 751-761 (2016).
41. Madakashira, B.P. & Sadler, K.C. DNA Methylation, Nuclear Organization, and Cancer. Front Genet 8, 76 (2017).
42. Spielmann, M., Lupianez, D.G. & Mundlos, S. Structural variation in the 3D genome. Nat Rev Genet 19, 453-467 (2018).
43. Johnson, D.S., Mortazavi, A., Myers, R.M. & Wold, B. Genome-wide mapping of in vivo protein- DNA interactions. Science 316, 1497-502 (2007).
44. Buenrostro, J.D., Giresi, P.G., Zaba, L.C., Chang, H.Y. & Greenleaf, W.J. Transposition of native chromatin for fast and sensitive epigenomic profiling of open chromatin, DNA-binding proteins and nucleosome position. Nat Methods 10, 1213-8 (2013).
45. Boyle, A.P. et al. High-resolution mapping and characterization of open chromatin across the genome. Cell 132, 311-22 (2008).
46. Schones, D.E. et al. Dynamic regulation of nucleosome positioning in the human genome. Cell 132, 887-98 (2008).
47. Ahringer, J. & Gasser, S.M. Repressive Chromatin in Caenorhabditis elegans: Establishment, Composition, and Function. Genetics 208, 491-511 (2018).
48. Towbin, B.D. et al. Step-wise methylation of histone H3K9 positions heterochromatin at the nuclear periphery. Cell 150, 934-47 (2012).
49. Zhang, H. et al. Loss of H3K9me3 Correlates with ATM Activation and Histone H2AX Phosphorylation Deficiencies in Hutchinson-Gilford Progeria Syndrome. PLoS One 11, e0167454 (2016).
50. Gonzalez-Sandoval, A. et al. Perinuclear Anchoring of H3K9-Methylated Chromatin Stabilizes Induced Cell Fate in C. elegans Embryos. Cell 163, 1333-47 (2015).
51. Harr, J.C., Gonzalez-Sandoval, A. & Gasser, S.M. Histones and histone modifications in perinuclear chromatin anchoring: from yeast to man. EMBO Rep 17, 139-55 (2016).
52. Teytelman, L., Thurtle, D.M., Rine, J. & van Oudenaarden, A. Highly expressed loci are vulnerable to misleading ChIP localization of multiple unrelated proteins. Proc Natl Acad Sci U S A 110, 18602-7 (2013).
53. Waldminghaus, T. & Skarstad, K. ChIP on Chip: surprising results are often artifacts. BMC Genomics 11, 414 (2010).
54. Dimri, G.P. et al. A biomarker that identifies senescent human cells in culture and in aging skin in vivo. Proc Natl Acad Sci U S A 92, 9363-7 (1995).
55. Gregoretti, F., Cesarini, E., Lanzuolo, C., Oliva, G. & Antonelli, L. An Automatic Segmentation Method Combining an Active Contour Model and a Classification Technique for Detecting Polycomb-group Proteinsin High-Throughput Microscopy Images. Methods Mol Biol 1480, 181- 97 (2016).
56. Goldstein, T., Bresson, X. & Osher, S. Geometric Applications of the Split Bregman Method: Segmentation and Surface Reconstruction. Journal of Scientific Computing 45, 272-293 (2010).
57. Ball, G. & Hall, D. ISODATA: A novel method of data analysis and pattern classification. (Stanford Research Institute, Menlo Park, 1965).
58. Li, H. & Durbin, R. Fast and accurate short read alignment with Burrows-Wheeler transform. Bioinformatics 25, 1754-60 (2009).
59. Li, H. et al. The Sequence Alignment/Map format and SAMtools. Bioinformatics 25, 2078-9 (2009).
60. Tischler, G. & Leonard, S. biobambam: tools for read pair collation based algorithms on BAM files. Source Code for Biology and Medicine 9, 13 (2014).
61. Quinlan, A.R. & Hall, I.M. BEDTools: a flexible suite of utilities for comparing genomic features. Bioinformatics 26, 841-2 (2010).
62. Bolger, A.M., Lohse, M. & Usadel, B. Trimmomatic: a flexible trimmer for Illumina sequence data. Bioinformatics 30, 2114-20 (2014).
63. Li, H. Toward better understanding of artifacts in variant calling from high-coverage samples. Bioinformatics 30, 2843-51 (2014).
64. Kharchenko, P.V., Tolstorukov, M.Y. & Park, P.J. Design and analysis of ChIP-seq experiments for DNA-binding proteins. Nat Biotechnol 26, 1351-9 (2008).
65. Kent, W.J., Zweig, A.S., Barber, G., Hinrichs, A.S. & Karolchik, D. BigWig and BigBed: enabling browsing of large distributed datasets. Bioinformatics 26, 2204-7 (2010).
66. Hahne, F. & Ivanek, R. Visualizing Genomic Data Using Gviz and Bioconductor. Methods Mol Biol 1418, 335-51 (2016).
67. Gel, B. & Serra, E. karyoploteR: an R/Bioconductor package to plot customizable genomes displaying arbitrary data. Bioinformatics 33, 3088-3090 (2017).
68. Wickham, H. ggplot2: Elegant Graphics for Data Analysis., (2016).
69. Nakamura, A., Osonoi, T. & Terauchi, Y. Relationship between urinary sodium excretion and pioglitazone-induced edema. J Diabetes Investig 1, 208-11 (2010).
70. Bray, N.L., Pimentel, H., Melsted, P. & Pachter, L. Near-optimal probabilistic RNA-seq quantification. Nat Biotechnol 34, 525-7 (2016).
71. Pimentel, H., Bray, N.L., Puente, S., Melsted, P. & Pachter, L. Differential analysis of RNA-seq incorporating quantification uncertainty. Nat Methods 14, 687-690 (2017).
72. Yi, L., Pimentel, H., Bray, N.L. & Pachter, L. Gene-level differential analysis at transcript-level resolution. Genome Biol 19, 53 (2018).

## Claims

1. An in vitro method for analyzing nucleic acids, the method comprising:
a) providing at least one isolated cell that comprises genetic material and
b) performing a salt-based sequential fractionation on genetic material to separate genomic regions on the basis of their accessibility and obtain chromatin fractions and
c) extracting the nucleic acids from the above chromatin fractions and
d) optionally sonicating the extracted nucleic acids such that chromatin is fragmented to have comparable profile of different fractions and
e) sequencing the extracted nucleic acids of step c) or the sonicated nucleic acid of step d), wherein the nucleic acid is DNA
and step b) comprises obtaining the following fractions: a fraction enriched of soluble nuclear and cytoplasmic proteins ("S1"), a fraction enriched in chromatin binding protein ("S2"), a fraction enriched in histones and histone bound proteins ("S3"), a fraction enriched in nucleo-skeleton and membrane associated proteins ("S4") and
after DNA extraction, the fraction S2 is sub fractionated in two sub fractions and one sub fraction comprising smaller nucleic acid fragments of about 100-200bp ("S2small" or "S2s") is directly isolated while the other sub fraction ("S2larger" or "S21") is subjected to the sonication of step d).

2. An in vitro method for analyzing nucleic acids, the method comprising:
a) providing at least one isolated cell that comprises genetic material and
b) performing a salt-based sequential fractionation on genetic material to separate genomic regions on the basis of their accessibility and obtain chromatin fractions and
c) extracting the nucleic acids from the above chromatin fractions and
d) sequencing the extracted nucleic acids of step c),
wherein the nucleic acid is RNA.

3. The method according to claim 2 wherein step b) comprises obtaining the following fractions: a fraction enriched of soluble nuclear and cytoplasmic proteins ("S1"), a fraction enriched in chromatin binding protein ("S2"), a fraction enriched in histones and histone bound proteins ("S3"), a fraction enriched in nucleo-skeleton and membrane associated proteins ("S4").

4. The method according to any one of previous claims, wherein the cell of step a) is previously obtained by:
- collecting cell removed from the culture substrate, preferably by trypsinization or
- digesting fragments of tissue, preferably in a dispase/collagenase buffer, in order to obtain a cell suspension and by filtering said suspension and collecting filtered cells.

5. The method according to any one of previous claims, wherein the at least one cell of step a) is a population of cells comprising 5×10⁴-50×10⁵, preferably 5-50×10⁵ cells.

6. The method according to any one of previous claims, wherein step b) comprises the following steps:
- extraction and solubilization of proteins from the cell to obtain the S1 fraction;
- nucleic acid digestion to obtain the S2 fraction;
- high salt extraction to obtain the S3 fraction and
- urea extraction to obtain S4 fraction and optionally
- sub fractioning S2 fraction into at least two fractions.

7. The method according to any one of previous claims wherein step b) comprises the following steps:
i. permeabilization of cell membranes and extractions of proteins from the cell to solubilize cytosolic and nuclear soluble proteins to obtain a solution, centrifugation of the obtained solution to separate cytoskeletal frameworks from soluble proteins thus obtaining a supernatatant and a pellet and collection of the obtained supernatant thus obtaining fraction S1,
ii. resuspension of the pellet obtained in step i., incubation of the suspended pellet with a nucleic acid cleaving agent to digest the nucleic acid, addition of a buffer able to stop the digestion, centrifugation to obtain a supernatant and a pellet and collection of the supernatant thus obtaining fraction S2;
iii. resuspension of the pellet obtained in step ii. in a buffer removing the histones from nucleic acid, centrifugation to obtain a supernatant and a pellet and collection of the supernatant thus obtaining fraction S3;
iv. resuspension of the pellet obtained in step iii. in a buffer removing the histones from nucleic acid, centrifugation to obtain a supernatant and a pellet, suspension of the pellet in a solution able to dissolve cellular membranes releasing membrane associated proteins, preferably said solution being urea, collection of the supernatant thus obtaining fraction S4.

8. The method according to claim 7 wherein:
step i. is carried out by incubation of the cell in a cytoskeleton buffer supplemented with a nonionic detergent, more preferably selected from the group of Triton X- 100, NP40, Tween20 and/or wherein step ii. comprises:
- washing the pellet obtained after centrifugation of i. in a cytoskeleton Triton buffer, centrifugation of the obtained suspension, collection and resuspension of the obtained pellet in a cytoskeleton buffer;
- addition of a DNA cleaving agent under conditions such that the agent cleaves the genomic DNA in the cell to solubilizes chromatin;
- addition of ammonium sulphate to obtain a solution;
- centrifugation of the solution obtained and collection of the obtained supernatant to have S2 fraction; and/or wherein
step iii. comprises:
- washing the obtained pellet in CSK buffer to obtain a solution;
- centrifugation of the obtained solution to obtain a supernatant and a pellet;
- salt extraction of the pellet, preferably by resuspension in CSK NaCl buffer, more preferably 2M NaCl in CSK;
- centrifugation of the solution obtained and collection of the obtained supernatant to have S3 fraction; and/or wherein
step iv. comprises:
- washing the obtained pellet in a buffer, preferably CSK NaCl, more preferably 2M NaCl in CSK, for at least two times;
- centrifugation of the obtained solution to obtain a pellet;
- solubilize the pellet in urea buffer to have S4 fraction.

9. The method according to claim 7 or 8 wherein the nucleic acid cleaving agent is an enzyme, preferably selected from a DNase and a restriction enzyme, more preferably RNase-free, preferably in a CSK buffer.

10. The method according to any one of claims 1 and 4-9 wherein the nucleic acid is DNA and in step d) the fractions S21, S3 and S4 are sonicated.

11. The method according to any one of previous claims, wherein the at least one cell is selected from the group of: eukaryotic cells, prokaryotic cells bacteria cells, plant cells, fungal cells, and animal cells, preferably human cell.

12. The method according to any of previous claims further comprising comparing the obtained sequence with a control for screening of patients affected by a pathology that alter genome conformation or for diagnosis of a pathology that alter genome conformation, preferably cancer or laminopathy, more preferably progeria.

13. An in vitro method of identifying cells suffering from a pathology by determining chromatin conformation of the target loci known to be associated with the disease by treating the group of cells according to the method of any one of claims 1-12.

14. Use of a kit for analyzing chromosomal nucleic acids for carrying out the method of any one of claims 1-13, the kit comprising at least one of the following: a high salt solution, reagents for isolating genetic material from cells, reagents for purifying genetic material, one or more restriction enzyme or a DNase, reagents for PCR amplifying target loci of interest, and reagents for sequencing the amplified target loci of interest using a DNA or RNA sequencing platform,.

## Patentansprüche

1. In-vitro-Verfahren zum Analysieren von Nukleinsäuren, wobei das Verfahren umfasst:
a) Bereitstellen von wenigstens einer isolierten Zelle, die genetisches Material umfasst, und
b) Durchführen einer salzbasierten sequenziellen Fraktionierung von genetischem Material, um genomische Bereiche auf Basis ihrer Zugänglichkeit abzutrennen und Chromatinfraktionen zu erhalten, und
c) Extrahieren der Nukleinsäuren aus den obigen Chromatinfraktionen und
d) gegebenenfalls Beschallen der extrahierten Nukleinsäuren, sodass das Chromatin fragmentiert wird, um ein vergleichbares Profil verschiedener Fraktionen zu erhalten, und
e) Sequenzieren der extrahierten Nukleinsäuren von Schritt c) oder der beschallten Nukleinsäure von Schritt d),
wobei die Nukleinsäure DNA ist
und wobei Schritt b) das Erhalten der folgenden Fraktionen umfasst: einer mit löslichen nukleären und zytoplasmatischen Proteinen angereicherte Fraktion ("S1"), einer mit dem Chromatinbindungsprotein angereicherten Fraktion ("S2"), einer mit Histonen und histongebundenen Proteinen angereicherten Fraktion ("S3"), einer mit nukleoskelett- und membranassoziierten Proteinen angereicherten Fraktion ("S4") und
wobei nach der DNA-Extraktion die Fraktion S2 in zwei Subfraktionen subfraktioniert wird und eine Subfraktion, die kleinere Nukleinsäurefragmente von etwa 100 bis 200 bp umfasst ("S2small" oder "S2s"), direkt isoliert wird, während die andere Subfraktion ("S2larger oder "S2l") der Beschallung von Schritt d) unterzogen wird.

2. In-vitro-Verfahren zum Analysieren von Nukleinsäuren, wobei das Verfahren umfasst:
a) Bereitstellen von wenigstens einer isolierten Zelle, die genetisches Material umfasst, und
b) Durchführen einer salzbasierten sequenziellen Fraktionierung von genetischem Material, um genomische Bereiche auf Basis ihrer Zugänglichkeit abzutrennen und Chromatinfraktionen zu erhalten, und
c) Extrahieren der Nukleinsäuren aus den obigen Chromatinfraktionen und
d) Sequenzieren der extrahierten Nukleinsäuren von Schritt c),
wobei die Nukleinsäure RNA ist.

3. Verfahren nach Anspruch 2, wobei Schritt b) das Erhalten der folgenden Fraktionen umfasst: einer mit löslichen nukleären und zytoplasmatischen Proteinen angereicherten Fraktion ("S1"), einer mit dem Chromatinbindungsprotein angereicherten Fraktion ("S2"), einer mit Histonen und histongebundenen Proteinen angereicherten Fraktion ("S3"), einer mit nukleoskelett- und membranassoziierten Proteinen angereicherten Fraktion ("S4").

4. Verfahren nach einem der vorhergehenden Ansprüche, wobei die Zelle von Schritt a) vorab erhalten wird durch:
- Sammeln der Zelle, die von dem Kultursubstrat entfernt wurde, vorzugsweise durch Trypsinierung, oder
- Verdauen von Gewebsfragmenten, vorzugsweise in einem Dispase/Collagenase-Puffer, um eine Zellsuspension zu erhalten, und Filtern der Suspension und Sammeln der gefilterten Zellen.

5. Verfahren nach einem der vorhergehenden Ansprüche, wobei die wenigstens eine Zelle von Schritt a) eine Population von Zellen ist, die 5 × 10⁴ bis 50 × 10⁵ Zellen, vorzugsweise 5 bis 50 × 10⁵ Zellen, umfasst.

6. Verfahren nach einem der vorhergehenden Ansprüche, wobei der Schritt b) die folgenden Schritte umfasst:
- Extraktion und Lösung von Proteinen von der Zelle zum Erhalt der Fraktion S1;
- Nukleinsäureverdauung, um die Fraktion S2 zu erhalten;
- Hochsalzextraktion, um die Fraktion S3 zu erhalten; und
- Harnstoffextraktion, um die Fraktion S4 zu erhalten; und gegebenenfalls
- Subfraktionierung der Fraktion S2 in wenigstens zwei Fraktionen.

7. Verfahren nach einem der vorhergehenden Ansprüche, wobei der Schritt b) die folgenden Schritte umfasst:
i. Permeabilisieren der Zellmembranen und Extrahieren von Proteinen aus der Zelle, um zytosolische und nukleäre lösliche Proteine zu lösen, um eine Lösung zu erhalten, Zentrifugieren der erhaltenen Lösung, um separate zytoskelettale Gerüste von den löslichen Proteinen zu trennen, wodurch ein Überstand und ein Pellet erhalten wird, und Sammeln des erhaltenen Überstands, wodurch die Fraktion S1 erhalten wird,
ii. Resuspendieren des in Schritt i. erhaltenen Pellets, Inkubieren des suspendierten Pellets mit einem Nukleinsäure-Spaltmittels, um die Nukleinsäure zu verdauen, Zugeben eines Puffers, der imstande ist, den Verdau zu stoppen, Zentrifugieren, um einen Überstand und ein Pellet zu erhalten, und Sammeln des Überstands, wodurch die Fraktion S2 erhalten wird;
iii. Resuspendieren des in Schritt ii. erhaltenen Pellets in einem Puffer, der die Histone von der Nukleinsäure entfernt, Zentrifugieren, um einen Überstand und ein Pellet zu erhalten, und Sammeln des Überstands, wodurch die Fraktion S3 erhalten wird;
iv. Resuspendieren des in Schritt iii. erhaltenen Pellets in einem Puffer, der die Histone von der Nukleinsäure entfernt, Zentrifugieren, um einen Überstand und ein Pellet zu erhalten, Suspendieren des Pellets in einer Lösung, die imstande ist, die Zellmembranen aufzulösen, wodurch membranassoziierte Proteine freigesetzt werden, wobei die Lösung vorzugsweise Harnstoff ist, Sammeln des Überstands, wodurch die Fraktion S4 erhalten wird.

8. Verfahren nach Anspruch 7, wobei:
der Schritt i. ausgeführt wird durch Inkubation der Zelle in einem Zytoskelett-Puffer, der mit einem nichtionischen Tensid supplementiert ist, das stärker bevorzugt aus der Gruppe aus Triton X-100, NP40, Tween20 ausgewählt wird, und/oder wobei
der Schritt ii. umfasst:
- Waschen des nach dem Zentrifugieren von i. erhaltenen Pellets in einem Zytoskelett-Triton-Puffer, Zentrifugieren der erhaltenen Suspension, Sammeln und Resuspendieren des erhaltenen Pellets in einem Zytoskelett-Puffer;
- Zugeben eines DNA-Spaltmittels unter derartigen Bedingungen, dass das Mittel die genomische DNA in der Zelle spaltet, um das Chromatin zu lösen;
- Zugeben von Ammoniumsulfat, um eine Lösung zu erhalten;
- Zentrifugieren der erhaltenen Lösung und Sammeln des erhaltenen Überstands, um die Fraktion S2 zu gewinnen; und/oder wobei
der Schritt iii. umfasst:
- Waschen des erhaltenen Pellets in einem CSK-Puffer, um eine Lösung zu erhalten;
- Zentrifugieren der erhaltenen Lösung, um einen Überstand und ein Pellet zu erhalten;
- Salzextraktion des Pellets, vorzugsweise durch Resuspendieren in einem CSK-NaCl-Puffer, stärker bevorzugt 2M NaCl in CSK;
- Zentrifugieren der erhaltenen Lösung und Sammeln des erhaltenen Überstands, um die Fraktion S3 zu gewinnen; und/oder wobei
der Schritt iv. umfasst:
- wenigstens zweimaliges Waschen des erhaltenen Pellets in einem Puffer, vorzugsweise CSK-NaCl, stärker bevorzugt 2M NaCl in CSK;
- Zentrifugieren der erhaltenen Lösung, um ein Pellet zu erhalten;
- Lösen des Pellets in einem Harnstoff-Puffer, um die Fraktion S4 zu gewinnen.

9. Verfahren nach Anspruch 7 oder 8, wobei das Nukleinsäure-Spaltmittel ein Enzym ist, das vorzugsweise aus einer DNase und einem Restriktionenzym, stärker bevorzugt RNase-frei, vorzugsweise in einem CSK-Puffer, ausgewählt ist.

10. Verfahren nach einem der Ansprüche 1 und 4 bis 9, wobei die Nukleinsäure DNA ist und in Schritt d) die Fraktionen S21, S3 und S4 beschallt werden.

11. Verfahren nach einem der vorhergehenden Ansprüche, wobei die wenigstens eine Zelle ausgewählt wird aus der Gruppe aus: eukaryontischen Zellen,
prokaryontischen Zellen, Bakterienzellen, Pflanzenzellen, Pilzzellen und Tierzellen, vorzugsweise menschlichen Zellen.

12. Verfahren nach einem der vorhergehenden Ansprüche, ferner umfassend das Vergleichen der erhaltenen Sequenz mit einer Kontrolle zum Screenen von Patienten, die von einer Krankheit betroffen sind, welche die Genomkonformation verändert, oder zum Diagnostizieren einer Krankheit, welche die Genomkonformation verändert, vorzugsweise Krebs oder eine Laminopathie, stärker bevorzugt Progerie.

13. In-vitro-Verfahren zum Identifizieren von an einer Krankheit leidenden Zellen durch das Bestimmen der Chromatin-Konformation der Zielorte, von denen bekannt ist, dass sie mit der Erkrankung assoziiert sind, indem die Gruppe von Zellen gemäß dem Verfahren nach einem der Ansprüche 1 bis 12 behandelt wird.

14. Verwendung eines Kits zum Analysieren von chromosomalen Nukleinsäuren zur Durchführung des Verfahrens nach einem der Ansprüche 1 bis 13, wobei das Kit wenigstens eines von Folgendem umfasst:
eine Hochsalzlösung, Reagenzien zum Isolieren von genetischem Material aus Zellen, Reagenzien zum Reinigen von genetischem Material, ein oder mehrere Restriktionsenzyme oder eine DNase, Reagenzien zur PCR-Amplifizierung der Zielorte von Interesse und
Reagenzien zum Sequenzieren der amplifizierten Zielorte von Interesse unter Verwendung einer DNA- oder RNA-Sequenzierungsplattform.

## Revendications

1. Procédé in vitro d'analyse d'acides nucléiques, le procédé comprenant :
a) la fourniture d'au moins une cellule isolée qui comprend du matériel génétique et
b) la réalisation d'un fractionnement séquentiel à base de sel sur le matériel génétique pour séparer les régions génomiques sur la base de leur accessibilité et obtenir des fractions de chromatine et
c) l'extraction des acides nucléiques des fractions de chromatine ci-dessus et
d) la sonication facultative des acides nucléiques extraits de telle sorte que la chromatine est fragmentée pour avoir un profil comparable de fractions différentes et
e) le séquençage des acides nucléiques extraits de l'étape c) ou l'acide nucléique soniqué de l'étape d),
dans lequel l'acide nucléique est de l'ADN
et l'étape b) comprend l'obtention des fractions suivantes : une fraction enrichie en protéines nucléaires et cytoplasmiques solubles (« S1 »), une fraction enrichie en protéines de liaison à la chromatine (« S2 »), une fraction enrichie en histones et en protéines liées aux histone (« S3 »), une fraction enrichie en nucléosquelette et protéines associées à la membrane (« S4 ») et
après l'extraction de l'ADN, la fraction S2 est sous-fractionnée en deux sous-fractions et une sous-fraction comprenant des fragments d'acide nucléique inférieurs d'environ 100 à 200 pb (« S2small » ou « S2s ») est directement isolée alors que l'autre sous-fraction (« S2larger » ou « S2l ») est soumise à la sonication de l'étape d).

2. Procédé in vitro d'analyse d'acides nucléiques, le procédé comprenant :
a) la fourniture d'au moins une cellule isolée qui comprend du matériau génétique et
b) la réalisation d'un fractionnement séquentiel à base de sel sur le matériau génétique pour séparer les régions génomiques sur la base de leur accessibilité et obtenir des fractions de chromatine et
c) l'extraction des acides nucléiques des fractions de chromatine ci-dessus et
d) le séquençage des acides nucléiques extraits de l'étape c),
dans lequel l'acide nucléique est de l'ARN.

3. Procédé selon la revendication 2 dans lequel l'étape b) comprend l'obtention des fractions suivantes : une fraction enrichie en protéines cytoplasmiques et nucléaires solubles (« S1 »), une fraction enrichie en protéines de liaison à la chromatine (« S2 »), une fraction enrichie en histones et en protéines de liaison aux histones (« S3 »), une fraction enrichie en nucléosquelette et protéines associées à la membrane (« S4 »).

4. Procédé selon l'une quelconque des revendications précédentes, dans lequel la cellule de l'étape a) est préalablement obtenue par :
- collecte de la cellule retirée du substrat cellulaire, de préférence par trypsinisation ou
- digestion de fragments de tissu, de préférence dans un tampon dipase/collagénase, de manière à obtenir une suspension cellulaire et par filtration de ladite suspension et collecte des cellules filtrées.

5. Procédé selon l'une quelconque des revendications précédentes, dans lequel l'au moins une cellule de l'étape a) est une population de cellules comprenant de 5 x 10⁴ à 50 x 10⁵ de préférence de 5 à 50 x 10⁵ cellules.

6. Procédé selon l'une quelconque des revendications précédentes, dans lequel l'étape b) comprend les étapes suivantes :
- extraction et solubilisation de protéines de la cellule pour obtenir la fraction S1 ;
- digestion de l'acide nucléique pour obtenir la fraction S2 ;
- extraction à haute teneur en sel pour obtenir la fraction S3 et
- extraction à l'urée pour obtenir la fraction S4 et facultativement
- sous-fractionnement de la fraction S2 en au moins deux fractions.

7. Procédé selon l'une quelconque des revendications précédentes dans lequel l'étape b) comprend les étapes suivantes :
i. la perméabilisation des membranes cellulaires et des extractions de protéines de la cellule pour solubiliser les protéines solubles nucléaires et cytosoliques pour obtenir une solution, la centrifugation de la solution obtenue pour séparer les structures cytosquelettiques des protéines solubles permettant ainsi d'obtenir un surnageant et un culot et la collecte du surnageant obtenu permettant ainsi d'obtenir la fraction S1,
ii. la remise en suspension du culot obtenu à l'étape i., l'incubation du culot en suspension avec un agent de clivage d'acide nucléique pour digérer l'acide nucléique, l'ajout d'un tampon capable d'arrêter la digestion, la centrifugation pour obtenir un surnageant et un culot et la collecte du surnageant permettant ainsi d'obtenir la fraction S2 ;
iii. la remise en suspension du culot obtenu à l'étape ii. dans un tampon retirant les histones de l'acide nucléique, la centrifugation pour obtenir un surnageant et un culot et la collecte du surnageant permettant ainsi d'obtenir la fraction S3 ;
iv. la remise en suspension du culot obtenu à l'étape iii. dans un tampon éliminant les histones de l'acide nucléique, la centrifugation pour obtenir un surnageant et un culot, la mise en suspension du culot dans une solution apte à dissoudre les membranes cellulaires libérant les protéines associées à la membrane, de préférence ladite solution étant l'urée, la collecte du surnageant permettant ainsi d'obtenir la fraction S4.

8. Procédé selon la revendication 7 dans lequel :
l'étape i. est réalisée par incubation de la cellule dans un tampon cytosquelette supplémenté d'un détergent non ionique, de manière davantage préférée sélectionné dans le groupe consistant en le Triton X - 100, NP40, Tween20 et/ou dans lequel
l'étape ii. comprend :
- le lavage du culot obtenu après la centrifugation de i. dans un tampon Triton cytosquelette, la centrifugation de la suspension obtenue, la collecte et la remise en suspension du culot obtenu dans un tampon cytosquelette ;
- l'ajout d'un agent de clivage de l'ADN dans des conditions telles que l'agent clive l'ADN génomique dans la cellule pour solubiliser la chromatine ;
- l'ajout de sulfate d'ammonium pour obtenir une solution ;
- la centrifugation la solution obtenue et la collecte du surnageant obtenu pour avoir une fraction S2 ; et/ou dans lequel
l'étape iii. comprend :
- le lavage du culot obtenu dans un tampon CSK pour obtenir une solution ;
- la centrifugation de la solution obtenue pour obtenir un surnageant et un culot ;
- l'extraction au sel du culot, de préférence par remise en suspension dans un tampon CSK NaCl, de manière davantage préféré NaCl 2M dans du CSK ;
- la centrifugation de la solution obtenue et la collecte du surnageant obtenu pour avoir une fraction S3 ; et/ou dans lequel
l'étape iv. comprend :
- le lavage du culot obtenu dans un tampon, de préférence CSK NaCl, de manière davantage préférée NaCl 2 M dans du CSK, au moins 2 fois ;
- la centrifugation de la solution obtenue pour obtenir un culot ;
- la solubilisation du culot dans un tampon urée pour obtenir une fraction S4.

9. Procédé selon la revendication 7 ou 8 dans lequel l'agent de clivage d'acide nucléique est une enzyme de préférence sélectionnée parmi une DNase et une enzyme de restriction, de manière davantage préférée dépourvu de RNase, de préférence dans un tampon CSK.

10. Procédé selon l'une quelconque des revendications 1 et 4 à 9 dans lequel l'acide nucléique est un ADN et à l'étape d) les fractions S21, S3 et S4 sont soniquées.

11. Procédé selon l'une quelconque des revendications précédentes, dans lequel l'au moins une cellule est sélectionnée dans le groupe consistant en : des cellules eucaryotes, des cellules procaryotes, des cellules bactériennes, des cellules végétales, des cellules fongiques, et des cellules animales, de préférence une cellule humaine.

12. Procédé selon l'une quelconque des revendications précédentes comprenant en outre la comparaison de la séquence obtenue avec un témoin pour le dépistage de patients affectés par une pathologie qui altère la conformation génomique ou pour le diagnostic d'une pathologie qui altère la conformation génomique, de préférence un cancer ou une laminopathie, de manière davantage préférée la progéria.

13. Procédé in vitro d'identification de cellules souffrant d'une pathologie par détermination de la conformation de la chromatine des locus cibles connus pour être associés à la maladie par traitement du groupe de cellules conformément au procédé selon l'une quelconque des revendications 1 à 12.

14. Utilisation d'un kit pour l'analyse d'acides nucléiques chromosomiques pour la mise en œuvre du procédé selon l'une quelconque des revendications 1 à 13, le kit comprenant au moins l'un des suivants :
une solution à haute teneur en sel, des réactifs pour l'isolement de matériel génétique de cellules, des réactifs pour la purification du matériel génétique,
une ou plusieurs enzymes de restriction ou une DNase,
des réactifs pour l'amplification par PCR des locus cibles d'intérêt, et des réactifs pour le séquençage des locus cibles amplifiés d'intérêt en utilisant une plateforme de séquençage d'ADN ou d'ARN.
